# EUROPEAN PATENT SPECIFICATION

(11) **EP 3 551 751 B1**
(45) Date of publication and mention of the grant of the patent: **02.12.2026**
(21) Application number: 17823051.2
(22) Date of filing: 04.12.2017
(51) Int. Cl.: C12N 9/02, A23L 27/30

(54) **KAURENOIC ACID HYDROXYLASES**
KAURENSÄUREHYDROXYLASEN
HYDROXYLASES D'ACIDE KAURENOIQUE

(30) Priority: 08.12.2016 EP 16202945
(43) Date of publication of application: 16.10.2019
(73) Proprietor: DSM IP Assets B.V., 6221 BE Maastricht (NL)
(72) Inventor: VAN LEEUWEN, Johannes, Gustaaf, Ernst, 6100 AA Echt (NL); ZWARTJENS, Priscilla, 6100 AA Echt (NL); BOER, Viktor, Marius, 6100 AA Echt (NL)
(74) Representative: dsm-firmenich IP
(86) International application number: PCT/EP2017/081390
(87) International publication number: WO 2018/104238

(56) References cited:
- EP-A1- 2 902 410
- EP-A2- 1 897 951
- WO-A1-2012/075030
- ANONYMOUS: "PREDICTED: cytochrome P450 714A2 [Camelina sativa] - Protein - NCBI", 29 November 2016 (2016-11-29), XP055895428, Retrieved from the Internet <URL:https://www.ncbi.nlm.nih.gov/protein/XP_010421338> [retrieved on 20220225]
- DATABASE UniProt [online] 29 October 2014 (2014-10-29), "SubName: Full=Uncharacterized protein {ECO:0000313|EMBL:KFK27759.1};", XP002771155, retrieved from EBI accession no. UNIPROT:A0A087GD07 Database accession no. A0A087GD07
- DATABASE UniProt [online] 29 April 2015 (2015-04-29), "SubName: Full=Uncharacterized protein {ECO:0000313|EnsemblPlants:Bo2g159140.1};", XP002771156, retrieved from EBI accession no. UNIPROT:A0A0D3AXT0 Database accession no. A0A0D3AXT0

## Description

### Field

The present disclosure relates to a polypeptide having kaurenoic acid 13-hydroxylase activity and to a nucleic acid comprising a sequence encoding such a polypeptide. The disclosure also relates to a nucleic acid construct comprising the nucleic acid and to an expression vector comprising the nucleic acid or nucleic acid construct. Further, the disclosure relates to a recombinant host comprising the nucleic acid, a nucleic acid construct or expression vector. The disclosure also relates to a process for the preparation of steviol or a steviol glycoside which comprises fermenting a recombinant host, to a fermentation broth obtainable by such a process.

### Background

The leaves of the perennial herb, *Stevia rebaudiana* Bert., accumulate quantities of intensely sweet compounds known as steviol glycosides. Whilst the biological function of these compounds is unclear, they have commercial significance as alternative high potency sweeteners.

These sweet steviol glycosides have functional and sensory properties that appear to be superior to those of many high potency sweeteners. In addition, studies suggest that stevioside can reduce blood glucose levels in Type II diabetics and can reduce blood pressure in mildly hypertensive patients.

Steviol glycosides accumulate in Stevia leaves where they may comprise from 10 to 20% of the leaf dry weight. Stevioside and rebaudioside A are both heat and pH stable and suitable for use in carbonated beverages and can be applied in many other foods. Stevioside is between 110 and 270 times sweeter than sucrose, rebaudioside A between 150 and 320 times sweeter than sucrose. In addition, rebaudioside D is also a high-potency diterpene glycoside sweetener which accumulates in Stevia leaves. It may be about 200 times sweeter than sucrose. Rebaudioside M is a further high-potency diterpene glycoside sweetener. It is present in trace amounts in certain stevia variety leaves, but has been suggested to have a superior taste profile.

Steviol glycosides have traditionally been extracted from the *Stevia* plant. In *Stevia,* (-)-kaurenoic acid, an intermediate in gibberellic acid (GA) biosynthesis, is converted into the tetracyclic diterpene steviol, which then proceeds through a multi-step glycosylation pathway to form the various steviol glycosides. However, yields may be variable and affected by agriculture and environmental conditions. Also, Stevia cultivation requires substantial land area, a long time prior to harvest, intensive labour and additional costs for the extraction and purification of the glycosides.

More recently, interest has grown in producing steviol glycosides using fermentative processes. WO2013/110673 and WO2015/007748 describe microorganisms that may be used to produce at least the steviol glycosides rebaudioside A and rebaudioside D.

Further improvement of such microorganisms is desirable in order that higher amounts of steviol glycosides may be produced and/or additional or new steviol glycosides and/or higher amounts of specific steviol glycosides and/or mixtures of steviol glycosides having desired ratios of different steviol glycosides.

### Summary

The invention is set out in the appended set of claims.

The present disclosure is based on the identification of new kaurenoic acid 13-hydroxylase (KAH) polypeptides, i.e. new polypeptides having KAH activity. These polypeptides may be used in the production of recombinant hosts suitable for the production of steviol and/or one or more steviol glycosides.

Such recombinant hosts may produce higher amounts of steviol glycosides and lower amount of non-desirable products as compared with recombinant hosts expressing a reference kaurenoic acid 13-hydroxylase. Production of higher amounts of steviol glycosides and/or lower amount of non-desirable products may make recovery of steviol glycosides easier. Also, a higher yield may be obtained.

Accordingly, the invention relates to a polypeptide having kaurenoic acid 13-hydroxylase activity, which polypeptide comprises an amino acid sequence which, when aligned with a kaurenoic acid 13-hydroxylase comprising the sequence set out in SEQ ID NO: 1 (the wild type KAH sequence from A. *thaliana)* or SEQ ID NO: 3 (KAH4_m4), comprises at least one substitution of an amino acid corresponding to any of amino acids at positions:
136, 248, 336 or 403, wherein the substitution to the amino acid corresponding to position 136 is a methionine, alanine, phenylalanine, tryptophan, glutamine, histidine or threonine,
said positions being defined with reference to SEQ ID NO: 1 or SEQ ID NO: 3 and wherein the polypeptide has a modified KAH activity as compared with the reference polypeptide having kaurenoic acid 13-hydroxylase activity according to SEQ ID NO: 3, wherein the modified activity leads to an increase of the ratio between the sum of steviol glycosides and the sum of kaurenoic acid-glycosides when the polypeptide with modified activity is overexpressed in a host cell as compared to the production level of an equivalent host cell which overexpresses the reference polypeptide, wherein the sum of steviol glycosides is the sum of the production level of steviol-13-monoside, steviol-19-monoside, steviol bioside, rubusoside, Rebaudioside B, stevioside, Rebaudioside A, Rebaudioside E, Rebaudioside D and Rebaudioside M, wherein the sum of KA-glycosides is the sum of the production level of KA, KA-19-monoglucoside, KA-19-diglucoside and KA-19-triglucoside, wherein the positions in the polypeptide having kaurenoic acid 13-hydroxylase activity corresponding to any amino acids at position 136, 248, 336 or 403, are identified by aligning the amino acid sequence of the polypeptide with kaurenoic acid 13-hydroxylase activity with the amino acid sequence set out in SEQ ID NO: 1 or SEQ ID NO: 3 using the EMBOSS Needle alignment method, using EBLOSUM62 as a substitution matrix, with a gap-open penalty of 10 and a gap extension penalty of 0.5. and wherein the polypeptide comprises an amino acid sequence having at least 90%, at least 95%, at least 97%, at least 98% or at least 99% sequence identity with SEQ ID NO: 1 or SEQ ID NO: 3.

The invention also relates to:
- a nucleic acid comprising a sequence encoding a polypeptide having kaurenoic acid 13-hydroxylase activity according to the invention;
- a recombinant host comprising a nucleic acid according to the invention;
- a process for the preparation of steviol or a steviol glycoside which comprises fermenting a recombinant host according to the invention in a suitable fermentation medium and, optionally, recovering the steviol or steviol glycoside;
- a fermentation broth comprising a steviol glycoside and the recombinant host cell according to the invention.

### Brief description of the drawings

Figure 1 set out a schematic diagram of some, but not all, of the potential pathways leading to biosynthesis of steviol glycosides.
Figure 2 sets out the plasmid map for genes encoding polypeptides having KAH activity cloned into a vector containing the INT3 integration flanks (which allow homologous recombination in *Y. lipolytica),* and promotor-orf-terminator for KAH4 and HygB (encoding for resistance against hygromycin).

### Description of the sequence listing

SEQ ID NO: 1 sets out the amino acid sequence of a kaurenoic acid 13-hydroxylase polypeptide from *Arabidopsis thaliana.*
SEQ ID NO: 2 sets out the nucleotide sequence encoding a kaurenoic acid 13-hydroxylase polypeptide from *Arabidopsis thaliana,* codon-pair optimized for expression in *Yarrowia lipolytica.*
SEQ ID NO: 3 sets out the amino acid sequence of the KAH4_m4 polypeptide.
SEQ ID NO: 4 sets out the nucleotide sequence encoding the KAH4_m4 polypeptide, codon-pair optimized for expression in *Yarrowia lipolytica.*
SEQ ID NOs: 5 to 14 are described in Table 1.
SEQ ID NO: 15 sets out the nucleotide sequence encoding a hydroxymethylglutaryl-CoA reductase polypeptide from *Yarrowia lipolytica,* codon-pair optimized for expression in *Yarrowia lipolytica.*
SEQ ID NO: 16 sets out the nucleotide sequence encoding a geranylgeranyl diphosphate synthase polypeptide from *Yarrowia lipolytica,* codon-pair optimized for expression in *Yarrowia lipolytica.*
SEQ ID NO: 17 sets out the nucleotide sequence encoding a geranylgeranyl diphosphate synthase polypeptide from *Mucor circenelloides,* codon optimized for expression in *Yarrowia lipolytica.*
SEQ ID NO: 18 sets out the nucleotide sequence encoding a copalyl pyrophosphate synthase polypeptide from *Stevia rebaudiana,* codon-pair optimized for expression in *Yarrowia lipolytica.*
SEQ ID NO: 19 sets out the nucleotide sequence encoding a kaurene synthase polypeptide from *Stevia rebaudiana,* codon-pair optimized for expression in *Yarrowia lipolytica.*
SEQ ID NO: 20 sets out the nucleotide sequence encoding a kaurene oxidase polypeptide from *Giberella fujikuroi,* codon-pair optimized for expression in *Yarrowia lipolytica.*
SEQ ID NO: 21 sets out the nucleotide sequence encoding a cytochrome P450 reductase polypeptide from *Arabidopsis thaliana,* codon-pair optimized for expression in *Yarrowia lipolytica.*
SEQ ID NO: 22 sets out the nucleotide sequence encoding a UDP-glucosyltransferase polypeptide from *Stevia rebaudiana,* codon-pair optimized for expression in *Yarrowia lipolytica.*
SEQ ID NO: 23 sets out the nucleotide sequence encoding a variant of UDP-glucosyltransferase polypeptide from *Stevia rebaudiana,* codon-pair optimized for expression in *Yarrowia lipolytica.*
SEQ ID NO: 24 sets out the nucleotide sequence encoding a UDP-glucosyltransferase polypeptide from *Stevia rebaudiana,* codon-pair optimized for expression in *Yarrowia lipolytica.*
SEQ ID NO: 25 sets out the nucleotide sequence encoding a UDP-glucosyltransferase polypeptide from *Stevia rebaudiana,* codon-pair optimized for expression in *Yarrowia lipolytica.*

### Detailed description

Throughout the present specification and the accompanying claims, the words "comprise", "include" and "having" and variations such as "comprises", "comprising", "includes" and "including" are to be interpreted inclusively. That is, these words are intended to convey the possible inclusion of other elements or integers not specifically recited, where the context allows.

The articles "a" and "an" are used herein to refer to one or to more than one (i.e. to one or at least one) of the grammatical object of the article. By way of example, "an element" may mean one element or more than one element.

According to the disclosure, there is thus provided a polypeptide having kaurenoic acid 13-hydroxylase activity. A polypeptide of the disclosure has kaurenoic acid 13-hydroxylase activity. Kaurenoic acid 13-hydroxylase activity is the activity of hydroxylation of (-)- kaurenoic acid at the C-13 position to form steviol.

"Rebaudioside" herein may be abbreviated to "Reb" or "reb" or the like.

Thus, for the purposes of the disclosure, a polypeptide having kaurenoic acid 13-hydroxylase activity may be one which is capable of catalysing or partially catalyzing the formation of steviol (ent-kaur-16-en-13-ol-19-oic acid) from ent-kaurenoic acid. For the purposes of the disclosure therefore, a polypeptide may be one having kaurenoic acid 13-hydroxylase activity is one which is capable of catalysing or partially catalyzing the formation of steviol (entkaur-16-en-13-ol-19-oic acid) using NADPH and O₂.

Such activity may also be referred to as ent-ka 13-hydroxylase activity or ent-kaurenoic acid 13-hydroxylase activity.

A polypeptide according to the disclosure has modified kaurenoic acid 13-hydroxylase activity as compared with a reference polypeptide having kaurenoic acid 13-hydroxylase activity according to SEQ ID NO: 3.

A polypeptide according to the disclosure may be a non-naturally occurring polypeptide.

Herein, polypeptides of the disclosure may be referred to as a "kaurenoic acid 13-hydroxylase" enzyme or polypeptide, "kaurenoic acid hydroxylase" enzyme or polypeptide, "KAH" enzyme or polypeptide or the like.

A KAH polypeptide of the disclosure comprises an amino acid sequence having at least 90% sequence identity with SEQ ID NO: 1 or SEQ ID NO; 2. Such a KAH polypeptide has one or more substitution or sets of substitutions selected from a position corresponding to
136, 248, 336 or 403, wherein the substitution to the amino acid corresponding to position 136 is a methionine, alanine, phenylalanine, tryptophan, glutamine, histidine or threonine,
said positions being defined with reference to SEQ ID NO: 1 or SEQ ID NO: 3.

An amino acid position corresponding to one of the positions 136, 248, 336 or 403, said position being defined with reference to SEQ ID NO: 1 or SEQ ID NO: 3 is a position which is identified in the KAH polypeptide sequence when the latter is aligned with the amino acid sequence set out in SEQ ID NO: 1 or 3 by a suitable sequence alignment method. A suitable sequence alignment method is a method which allows comparison of the sequences with each other and identifications of the positions in the amino acid sequence of KAH polypeptide wherein either the same amino acid is present (identical position), or another amino acid is present (substitution), or one or more extra amino acids are present (insertion or extension) or no amino acid is present (deletion or truncation) if compared with the amino acid sequence set out in SEQ ID NO: 1 or 3.

The suitable method allowing comparison of two amino acid sequence is a Global Pairwise Sequence Alignment method. The Global Pairwise Sequence Alignment method used herein is the EMBOSS Needle method based on the Needleman-Wunsch alignment algorithm (aiming at finding the optimum alignment (including gaps) of the two sequences along their entire length) (Needleman, S. B. and Wunsch, C. D. (1970) J. Mol. Biol. 48, 443-453) as described herein. The amino acid sequence is aligned with the amino acid sequence set out in SEQ ID NO: 1 or 3 using the EMBOSS Needle alignment method using EBLOSUM62 as a substitution matrix, with a gap-open penalty of 10 and a gap extension penalty of 0.5.

The positions in the polypeptide having KAH activity corresponding to any amino acids at position 136, 248, 336 or 403, said position being defined with reference to SEQ ID NO: 1 or SEQ ID NO: 3, are identified by aligning the amino acid sequence of the polypeptide with KAH activity of the disclosure with the amino acid sequence set out in SEQ ID NO: 1 or 3 using the EMBOSS Needle alignment method , using the NEEDLE program from the EMBOSS package, using EBLOSUM62 as a substitution matrix, with a gap-open penalty of 10 and a gap extension penalty of 0.5.

A KAH of the disclosure retains KAH activity. A KAH of the disclosure is capable of catalysing the reaction set out above, albeit with a modified activity as compared with a reference polypeptide.

A KAH polypeptide comprises an amino acid sequence that has one or more substitution, deletion and/or insertion of an amino acid as compared to the reference polypeptide and/or one or more truncations as compared to the reference polypeptide. A KAH polypeptide comprises one or more of the substitutions described herein.

A polypeptide having KAH activity, as set out herein, which polypeptide comprises an amino acid sequence which, when aligned with the KAH comprising the sequence set out in SEQ ID NO: 1 or SEQ ID NO: 3, comprises at least one substitution of an amino acid corresponding to any of amino acids
136, 248, 336 or 403, wherein the substitution to the amino acid corresponding to position 136 is a methionine, alanine, phenylalanine, tryptophan, glutamine, histidine or threonine,
said positions being defined with reference to SEQ ID NO: 1 or SEQ ID NO: 3 and wherein the KAH, has a modified KAH activity as compared with the reference polypeptide having kaurenoic acid 13-hydroxylase activity according to SEQ ID NO: 3.

Substitution of an amino acid is intended to indicate that the amino acid residue at the specified position is replaced with a different amino acid.

Thus, the amino acid present at one or more of the said positions will be replaced with a different amino acid than appears at that position in the reference sequence (the positions being defined with reference to SEQ ID NO: 1 or SEQ ID NO: 3).

A KAH of the disclosure may comprise one of the substitutions set out above, or may comprise any combination of two, three or four of them.

A KAH polypeptide of the disclosure is one wherein:
(i) a methionine (M), alanine (A), phenylalanine (F), tryptophan (W), glutamine (Q), histidine (H) or a threonine (T) is present at a position corresponding to 136;
and may be one wherein:
(ii) an asparagine (N), glutamine (Q), threonine (T), glycine (G), alanine (A), isoleucine (I), valine (V), phenylalanine (F) or proline (P) is present at a position corresponding 248;
(iii) a serine (S) an alanine (A) or isoleucine (I) is present at a position corresponding to 336; and/or
(iv) a glycine (G), leucine (L), valine (V), alanine (A), methionine (M) or serine (S) is present at a position corresponding to 403,
said positions being defined with reference to SEQ ID NO: 1 or SEQ ID NO: 3.

Any combination of two, three or four of the above defined substitutions may be used to define a KAH of the disclosure.

A KAH polypeptide of the disclosure may preferably be one wherein:
(i) a methionine (M) is present at a position corresponding to 136;
(ii) an asparagine (N) is present at a position corresponding 248;
(iii) a serine (S) is present at a position corresponding to 336; and/or
(iv) a glycine (G) is present at a position corresponding to 403,
said positions being defined with reference to SEQ ID NO: 1 or SEQ ID NO: 3.

Any combination of the above defined substitutions may be used to define a KAH of the disclosure.

Thus, a KAH polypeptide of the disclosure may comprise an amino acid sequence which, when aligned with the KAH comprising the sequence set out in SEQ ID NO: 1 or SEQ ID NO: 3, comprises at least the following substitutions of an amino acid corresponding to any of amino acids:
136 and 248
136 and 336
136 and 403
248 and 336
248 and 403
336 and 403
136, 248 and 403
136, 336 and 403
136, 248 and 336
248, 336 and 403 or
136, 248, 336 and 403,
said positions being defined with reference to SEQ ID NO: 1 or SEQ ID NO: 3.

Thus, a KAH polypeptide of the disclosure may comprise an amino acid sequence which, when aligned with the KAH comprising the sequence set out in SEQ ID NO: 1 or SEQ ID NO: 3, comprises at least the following substitutions of an amino acid corresponding to any of amino acids:
a) a methionine (M), or alanine (A), or phenylalanine (F), or tryptophan (W), or glutamine (Q), or histidine (H) or a threonine (T) present at a position corresponding to position 136 and an asparagine (N), or glutamine (Q), or threonine (T), or glycine (G), or alanine (A), or isoleucine (I), or valine (V), or phenylalanine (F) or proline (P) present at a position corresponding position 248;
b) a methionine (M), or alanine (A), phenylalanine (F), or tryptophan (W), or glutamine (Q), or histidine (H) or a threonine (T) is present at a position corresponding to 136 and a serine (S), or an alanine (A), or isoleucine (I) is present at a position corresponding to position 336;
c) a methionine (M), or alanine (A), phenylalanine (F), or tryptophan (W), or glutamine (Q), or histidine (H) or a threonine (T) is present at a position corresponding to 136 and a glycine (G), or leucine (L), or valine (V), or alanine (A), or methionine (M) or serine (S) is present at a position corresponding to position 403;
d) an asparagine (N), or glutamine (Q), or threonine (T), or glycine (G), or alanine (A), or isoleucine (I), or valine (V), or phenylalanine (F) or proline (P) present at a position corresponding position 248 and a serine (S), or an alanine (A), or isoleucine (I) is present at a position corresponding to position 336;
e) an asparagine (N), or glutamine (Q), or threonine (T), or glycine (G), or alanine (A), or isoleucine (I), or valine (V), or phenylalanine (F) or proline (P) present at a position corresponding position 248 and a glycine (G), or leucine (L), or valine (V), or alanine (A), or methionine (M) or serine (S) is present at a position corresponding to position 403;
f) a serine (S), or an alanine (A), or isoleucine (I) is present at a position corresponding to position 336 and a glycine (G), or leucine (L), or valine (V), or alanine (A), or methionine (M) or serine (S) is present at a position corresponding to position 403;
g) a methionine (M), or valine (V), or alanine (A), phenylalanine (F), or tryptophan (W), or glutamine (Q), or histidine (H) or a threonine (T) is present at a position corresponding to 136, an asparagine (N), or glutamine (Q), or threonine (T), or glycine (G), or alanine (A), or isoleucine (I), or valine (V), or phenylalanine (F) or proline (P) present at a position corresponding position 248 and a glycine (G), or leucine (L), or valine (V), or alanine (A), or methionine (M) or serine (S) is present at a position corresponding to position 403;
h) a methionine (M), or alanine (A), phenylalanine (F), or tryptophan (W), or glutamine (Q), or histidine (H) or a threonine (T) is present at a position corresponding to 136, a serine (S), or an alanine (A), or isoleucine (I) is present at a position corresponding to position 336 and a glycine (G), or leucine (L), or valine (V), or alanine (A), or methionine (M) or serine (S) is present at a position corresponding to position 403;
i) a methionine (M), or alanine (A), phenylalanine (F), or tryptophan (W), or glutamine (Q), or histidine (H) or a threonine (T) is present at a position corresponding to 136, an asparagine (N), or glutamine (Q), or threonine (T), or glycine (G), or alanine (A), or isoleucine (I), or valine (V), or phenylalanine (F) or proline (P) present at a position corresponding position 248 and a serine (S), or an alanine (A), or isoleucine (I) is present at a position corresponding to position 336;
j) an asparagine (N), or glutamine (Q), or threonine (T), or glycine (G), or alanine (A), or isoleucine (I), or valine (V), or phenylalanine (F) or proline (P) present at a position corresponding position 248, a serine (S), or an alanine (A), or isoleucine (I) is present at a position corresponding to position 336 and a glycine (G), or leucine (L), or valine (V), or alanine (A), or methionine (M) or serine (S) is present at a position corresponding to position 403 or
k) a methionine (M), or alanine (A), phenylalanine (F), or tryptophan (W), or glutamine (Q), or histidine (H) or a threonine (T) is present at a position corresponding to 136, an asparagine (N), or glutamine (Q), or threonine (T), or glycine (G), or alanine (A), or isoleucine (I), or valine (V), or phenylalanine (F) or proline (P) present at a position corresponding position 248, a serine (S), or an alanine (A), or isoleucine (I) is present at a position corresponding to position 336 and a glycine (G), or leucine (L), or valine (V), or alanine (A), or methionine (M) or serine (S) is present at a position corresponding to position 403,
said positions being defined with reference to SEQ ID NO: 1 or SEQ ID NO: 3.

A KAH polypeptide of the disclosure may comprise additional substitutions other than one or more of the five substitutions positions defined above, for example, one or more additional substitutions, additions or deletions.

A KAH of the disclosure may comprise a combination of different types of modification of this sort. A KAH may comprise one, two, three, four, at least 5, at least 10, at least 15, at least 20, at least 25, at least 30 or more such modifications (which may all be of the same type or may be different types of modification). Typically, the additional modifications may be substitutions.

A KAH polypeptide of the disclosure may comprise the amino acid sequence set out in SEQ ID NO: 3. However, a KAH polypeptide may comprise any combination of substitutions at positions 136, 248, 336 or 403, wherein the substitution to the amino acid corresponding to position 136 is a methionine, alanine, phenylalanine, tryptophan, glutamine, histidine or threonine, said positions being defined with reference to a suitable reference sequence such as that set out in SEQ ID NO: 1 or SEQ ID NO: 3.

A host cell may comprise nucleic acids encoding one, two, three, four, five or more KAHs of the disclosure. Such KAH polypeptides may be the same or different. A host cell may comprise a nucleic acid encoding the KAH of SEQ ID NO: 1 or SEQ ID NO: 3 and a nucleic acid encoding one or more KAHs of the disclosure. That is to say, a host may comprise a nucleic acid encoding the KAH of SEQ ID NO: 1 or SEQ ID NO: 3 and a nucleic acid encoding one or more KAHs of the disclosure, each of which may be present in a copy of one, two, three, four, five or more.

A KAH polypeptide according to the disclosure has a modified KAH activity in comparison to a reference polypeptide.

The modified activity is defined in terms of a change in ratio of the sum of steviol glycosides produced to the sum of kaurenoic acid-glycosides, wherein the ratio of the sum of steviol glycosides: the sum of kaurenoic acid-glycosides is increased when a KAH is overexpressed in a host cell as compared to the production level of an equivalent host cell which overexpresses a reference polypeptide, according to SEQ ID NO: 3.

A KAH may be capable of increasing production levels, for example by at least 5%, at least 10%, at least 25%, at least 50%, at least 100% or more. Production levels may be expressed in terms of g/L or mol/L (M), so an increase in the production level of steviol and/or steviol glycosides will be evident by higher level of production in terms of g/L or mol/L.

In the case of a non-desirable product, such as one or more kaurenoic acid glycosides, a KAH may be capable of decreasing production levels for example by at least 5%, at least 10%, at least 25%, at least 50% or more. A KAH may be capable of increasing this ratio, for example by at least 1%, at least 2%, at least 5%, at least 10%, at least 25%, at least 50%, at least 100% or more.

As set out above, this is defined in terms of an increase of the sum of steviol glycosides: the sum of kaurenoic acid-glycosides.

The word "polypeptide" is used herein for chains containing more than about seven amino acid residues. All polypeptide sequences herein are written from left to right and in the direction from amino terminus to carboxy terminus. The one-letter code of amino acids used herein is commonly known in the art and can be found in Sambrook, et al. (Molecular Cloning: A Laboratory Manual, 2nd ed. Cold Spring Harbor Laboratory, Cold Spring Harbor Laboratory Press, Cold Spring Harbor, NY, 1989).

A KAH polypeptide of the disclosure may be in isolated form, such as substantially isolated form. By "isolated" polypeptide or protein is intended a polypeptide or protein removed from its native environment. For example, recombinantly produced polypeptides and proteins expressed in host cells are considered isolated for the purpose of the disclosure as are recombinant polypeptides which have been substantially purified by any suitable technique. A KAH polypeptide according to the disclosure can be recovered and purified from recombinant cell cultures by methods known in the art.

KAH polypeptides of the present disclosure include products of chemical synthetic procedures, and products produced by recombinant techniques from a prokaryotic or eukaryotic host, including, for example, bacterial, yeast, higher plant, insect and mammalian cells. Depending upon the host employed in a recombinant production procedure, the polypeptides of the present disclosure may be glycosylated or may be non-glycosylated. In addition, polypeptides of the disclosure may also include an initial modified methionine residue, in some cases as a result of host-mediated processes.

The present disclosure provides polynucleotides which comprise sequence encoding a KAH polypeptide of the disclosure.

A nucleic acid molecule of the present disclosure can be generated using standard molecular biology techniques well known to those skilled in the art taken in combination with the sequence information provided herein. For example, using standard synthetic techniques, the required nucleic acid molecule may be generated by PCR or synthesized *de novo.* Such a synthetic process will typically be an automated process.

A nucleic acid of the disclosure may comprise one or more deletions, i.e. gaps, in comparison to a nucleic acid encoding a reference KAH. Such deletions/gaps may also be generated using site-directed mutagenesis using appropriate oligonucleotides. Techniques for generating such deletions are well known to those skilled in the art.

One aspect of the disclosure pertains to isolated nucleic acid molecules that encode a KAH polypeptide of the disclosure.

An "isolated nucleic acid" or "isolated polynucleotide" is a DNA or RNA that is not immediately contiguous with both of the coding sequences with which it is immediately contiguous (one on the 5' end and one on the 3' end) in the naturally occurring genome of the organism from which it is derived. Thus, in one embodiment, an isolated nucleic acid includes some or all of the 5' non-coding (e.g., promotor) sequences that are immediately contiguous to the coding sequence. The term therefore includes, for example, a recombinant DNA that is incorporated into a vector, into an autonomously replicating plasmid or virus, or into the genomic DNA of a prokaryote or eukaryote, or which exists as a separate molecule (e.g., a cDNA or a genomic DNA fragment produced by PCR or restriction endonuclease treatment) independent of other sequences. It also includes a recombinant DNA that is part of a hybrid gene encoding an additional polypeptide that is substantially free of cellular material, viral material, or culture medium (when produced by recombinant DNA techniques), or chemical precursors or other chemicals (when chemically synthesized). Moreover, an "isolated nucleic acid fragment" is a nucleic acid fragment that is not naturally occurring as a fragment and would not be found in the natural state.

As used herein, the terms "nucleic acid", "polynucleotide" or "nucleic acid molecule" are intended to include DNA molecules (e.g., cDNA or genomic DNA) and RNA molecules (e.g., mRNA) and analogs of the DNA or RNA generated using nucleotide analogs. The nucleic acid molecule can be single-stranded or double-stranded, but preferably is double-stranded DNA. The nucleic acid may be synthesized using oligonucleotide analogs or derivatives (e.g., inosine or phosphorothioate nucleotides). Such oligonucleotides can be used, for example, to prepare nucleic acids that have altered base-pairing abilities or increased resistance to nucleases.

The disclosure also relates to a nucleic acid construct comprising a nucleic acid sequence encoding a KAH polypeptide of the disclosure and, linked operably thereto, control sequences permitting expression of the nucleic acid sequence in a host cell. The nucleic acid construct may be incorporated into a vector, such as an expression vector and/or into a host cell in order to effect expression of the KAH polypeptide.

The term "nucleic acid construct" is herein referred to as a nucleic acid molecule, either single-or double-stranded, which is isolated from a naturally-occurring gene or, more typically, which has been modified to contain segments of nucleic acid which are combined and juxtaposed in a manner which would not otherwise exist in nature. The term nucleic acid construct is synonymous with the term "expression cassette" when the nucleic acid construct contains all the control sequences required for expression of a coding sequence, wherein said control sequences are operably linked to said coding sequence.

As used herein, the term "operably linked" refers to a linkage of polynucleotide elements (or coding sequences or nucleic acid sequence) in a functional relationship. A nucleic acid sequence is "operably linked" when it is placed into a functional relationship with another nucleic acid sequence. For instance, a promoter or enhancer is operably linked to a coding sequence if it affects the transcription of the coding sequence.

As used herein, the term "promoter" refers to a nucleic acid fragment that functions to control the transcription of one or more genes, located upstream with respect to the direction of transcription of the transcription initiation site of the gene, and is structurally identified by the presence of a binding site for DNA-dependent RNA polymerase, transcription initiation sites and any other DNA sequences known to one of skilled in the art. A "constitutive" promoter is a promoter that is active under most environmental and developmental conditions. An "inducible" promoter is a promoter that is active under environmental or developmental regulation.

A promoter that could be used to achieve the expression of a nucleotide sequence coding for an enzyme such as a KAH polypeptide or any other enzyme introduced in recombinant host of the disclosure, may be not native to a nucleotide sequence coding for the enzyme to be expressed, i.e. a promoter that is heterologous to the nucleotide sequence (coding sequence) to which it is operably linked. Preferably, the promoter is homologous, i.e. endogenous to the host cell.

Suitable promoters in this context include both constitutive and inducible natural promoters as well as engineered promoters, which are well known to the person skilled in the art. Suitable promoters in host cells may be GAL7, GAL10, or GAL 1, CYC1, HIS3, ADH1, PGL, PH05, GAPDH, ADC1, TRP1, URA3, LEU2, ENO, TPI, and AOX1. Other suitable promoters include PDC, GPD1, PGK1, TEF1, and TDH.

Usually a nucleotide sequence encoding an enzyme comprises a terminator. Any terminator, which is functional in a host cell, may be used in the present disclosure. Preferred terminators are obtained from natural genes of the host cell. Suitable terminator sequences are well known in the art. Preferably, such terminators are combined with mutations that prevent nonsense mediated mRNA decay in the host cell of the disclosure (see for example: Shirley et al., 2002, Genetics 161:1465-1482).

The disclosure further relates to a vector, preferably an expression vector, comprising a nucleic acid or a nucleic acid construct of the disclosure of the disclosure (i.e. comprising sequence encoding a KAH polypeptide of the disclosure).

In order to facilitate expression and/or translation of the KAH, the nucleic acid sequence encoding the KAH may be comprised in an expression vector such that the gene encoding the KAH is operably linked to the appropriate control sequences for expression and/or translation *in vitro,* or in a host cell of the disclosure. That is to say, the disclosure provides an expression vector comprising a nucleic acid or nucleic acid construct of the disclosure.

The expression vector may be any vector (e.g., a plasmid or virus), which can be conveniently subjected to recombinant DNA procedures and can bring about the expression of the polynucleotide encoding the KAH polypeptide. The choice of the vector will typically depend on the compatibility of the vector with the cell into which the vector is to be introduced. The vectors may be linear or closed circular plasmids. The vector may be an autonomously replicating vector, i. e., a vector, which exists as an extra-chromosomal entity, the replication of which is independent of chromosomal replication, e.g., a plasmid, an extra-chromosomal element, a mini-chromosome, or an artificial chromosome. If intended for use in a host cell of fungal origin, a suitable episomal nucleic acid construct may e.g. be based on the yeast 2µ or pKD1 plasmids (Gleer et al., 1991, Biotechnology 9: 968-975), or the AMA plasmids (Fierro et al., 1995, Curr Genet. 29:482-489).

Alternatively, the expression vector may be one which, when introduced into the host cell, is integrated into the genome and replicated together with the chromosome(s) into which it has been integrated. The integrative cloning vector may integrate at random or at a predetermined target locus in the chromosomes of the host cell. In a preferred embodiment of the disclosure, the integrative cloning vector comprises a DNA fragment, which is homologous to a DNA sequence in a predetermined target locus in the genome of host cell for targeting the integration of the cloning vector to this predetermined locus. In order to promote targeted integration, the cloning vector is preferably linearized prior to transformation of the cell. Linearization is preferably performed such that at least one but preferably either end of the cloning vector is flanked by sequences homologous to the target locus. The length of the homologous sequences flanking the target locus is preferably at least 20bp, at least 30 bp, at least 50 bp, at least 0.1 kb, at least 0.2 kb, at least 0.5 kb, at least 1 kb, at least 2 kb or longer. The efficiency of targeted integration into the genome of the host cell, i.e. integration in a predetermined target locus, is increased by augmented homologous recombination abilities of the host cell.

The homologous flanking DNA sequences in the cloning vector, which are homologous to the target locus, may be derived from a highly expressed locus meaning that they are derived from a gene, which is capable of high expression level in the host cell. A gene capable of high expression level, i.e. a highly expressed gene, is herein defined as a gene whose mRNA can make up at least 0.5% (w/w) of the total cellular mRNA, e.g. under induced conditions, or alternatively, a gene whose gene product can make up at least 1% (w/w) of the total cellular protein, or, in case of a secreted gene product, can be secreted to a level of at least 0.1 g/l. More typically, the target locus may be an intergenic location, so that a gene is not interrupted. Such a locus may also provide for high expression levels. Accordingly, the homologous flanking DNA sequences in the cloning vector may be homologous to an intergenic target locus
A nucleic acid construct or expression vector may be assembled in vivo in a host cell of the disclosure and, optionally, integrated into the genome of the cell in a single step (see, for example, WO2013/076280)
More than one copy of a nucleic acid construct or expression vector of the disclosure may be inserted into a host cell to increase production of the KAH polypeptide (over-expression) encoded by the nucleic acid sequence comprised within the nucleic acid construct. This can be done, preferably by integrating into its genome two or more copies of the nucleic acid, more preferably by targeting the integration of the nucleic acid to a locus defined as defined above.

It will be appreciated by those skilled in the art that the design of the expression vector can depend on such factors as the choice of the host cell to be transformed, the level of expression of protein desired, etc. The expression vectors of the disclosure can be introduced into host cells to thereby produce proteins or peptides, encoded by nucleic acids as described herein (e.g. a KAH of SEQ ID NO: 1 or SEQ ID NO: 3, for example a functional equivalent or fragment, or a fusion protein comprising one or more of such KAHs).

The nucleic acid constructs and vectors of the disclosure can be designed for expression of KAH polypeptides of the disclosure in a prokaryotic host cell or eukaryotic host cell.

A nucleic acid construct and/or expression vector of the disclosure can be introduced into prokaryotic or eukaryotic cells via conventional transformation or transfection techniques. As used herein, the terms "transformation" and "transfection" are intended to refer to a variety of art-recognized techniques for introducing foreign nucleic acid (e.g., DNA) into a host cell well known to those skilled in the art. Suitable methods for transforming or transfecting host cells can be found in Sambrook, et al. (Molecular Cloning: A Laboratory Manual, 2nd,ed. Cold Spring Harbor Laboratory, Cold Spring Harbor Laboratory Press, Cold Spring Harbor, NY, 1989), Davis et al., Basic Methods in Molecular Biology (1986) and other laboratory manuals.

"Functional equivalents" according to the disclosure are isolated nucleic acid fragments that encode a polypeptide that exhibits a particular function of a KAH of the disclosure as defined herein. Functional equivalents therefore also encompass biologically active fragments and are themselves encompassed within the term "a KAH" (or the like) of the disclosure.

Preferably, a functional equivalent of the disclosure comprises one or more of the substitutions described herein. However, a functional equivalent may comprise one or more modifications in addition to the substitutions described above.

Functional nucleic acid equivalents may typically contain silent mutations or mutations that do not alter the biological function of the encoded KAH polypeptide. Accordingly, the disclosure provides nucleic acid molecules encoding a KAH protein that contains changes in amino acid residues that are not essential for a particular biological activity, i.e. KAH activity.

Such functional equivalents of KAH proteins differ in amino acid sequence from the parent KAH sequence from which they are derived yet retain at least one biological activity thereof, preferably they retain at least KAH activity. The skilled person will recognise that changes can be introduced by mutation into the nucleotide sequences according to the disclosure thereby leading to changes in the amino acid sequence of the resulting protein without substantially altering the function of such a protein.

The isolated nucleic acid molecule comprises a nucleotide sequence encoding a protein, wherein the protein comprises an amino acid sequence having at least 90%, 95%, 96%, 97%, 98%, 99% identity with the reference amino acid sequence shown in SEQ ID NO: 1 or SEQ ID NO: 3.

Accordingly, a functional equivalent of a KAH of the disclosure is a protein which comprises an amino acid sequence having at least 90%, 95%, 96%, 97%, 98%, 99% or more identity to the reference polypeptide sequence shown in SEQ ID NO: 1 or SEQ ID NO: 3, and retains at least one functional activity of the parent KAH polypeptide.

A polypeptide of the disclosure having kaurenoic acid 13-hydroxylase activity may comprise an amino acid sequence having at least 95% sequence identity, at least about 96%, at least about 97%, at least about 98% or at least about 99% sequence identity to any one of SEQ ID NO: 5, SEQ ID NO: 7, SEQ ID NO: 9, SEQ ID NO: 11 or SEQ ID NO: 13.

A KAH polypeptide of the disclosure may have a sequence as defined in Table 1 or a substitution pattern as defined in Table 1 (in terms of position(s), if not precisely the same amino acid substitution).

KAH polypeptides of the disclosure may be identified e.g. by screening libraries of mutants, e.g. substitution mutants, of a suitable reference polypeptide. Candidate mutants may be screened on the basis of their ability to increase steviol or steviol glycoside production, when expressed in a host cell (in comparison with a corresponding host cell expressing the reference polypeptide).

Fragments of a nucleic acid according to the disclosure may comprise or consist of sequences not encoding functional polypeptides. Such nucleic acids may function as probes or primers for a PCR reaction.

Nucleic acids according to the disclosure irrespective of whether they encode functional or non-functional polypeptides can be used as hybridization probes or polymerase chain reaction (PCR) primers. Uses of the nucleic acid molecules of the present disclosure that do not encode a polypeptide having KAH activity include, inter alia, *(1) in situ* hybridization (e.g. FISH) to metaphase chromosomal spreads to provide precise chromosomal location of an KAH-encoding gene as described in Verma et al., Human Chromosomes: a Manual of Basic Techniques, Pergamon Press, New York (1988); (2) Northern blot analysis for detecting expression of KAH mRNA in specific tissues and/or cells; and (3) probes and primers that can be used as a diagnostic tool to analyse the presence of a nucleic acid hybridizable to such a probe or primer in a given biological (e.g. tissue) sample.

A KAH of the disclosure based on a given reference KAH enzyme can be obtained by the following standard procedure which is disclosed but not claimed:
- Mutagenesis (error-prone, doped oligo, spiked oligo) or synthesis of variants
- Transformation in, for example, *Y. lipolytica* or S. *cerevisiae*
- Cultivation of transformants, selection of transformants
- Expression in, for example, *Y. lipolytica* or S. *cerevisiae*
- Primary Screening, for example on the basis of steviol or steviol glycoside production
- Identification of an improved KAH

In another embodiment, the disclosure features host cells, e.g., transformed host cells or recombinant host cells that contain a nucleic acid, nucleic acid construct or vector of the disclosure. A "host cell" or "recombinant cell" according to the disclosure is typically a cell into which (or into an ancestor of which) has been introduced, by means of recombinant DNA techniques, a nucleic acid according to the disclosure, i.e. a nucleic acid encoding a KAH of the disclosure. In the context of the present disclosure a "host cell" according to the disclosure or a parent of said host cell may be any type of host cell.

Thus, a host cell of the disclosure may comprise a recombinant nucleic acid encoding one or more KAH polypeptides of the disclosure.

A host cell according to any one of the preceding claims wherein the host cell is a eukaryotic or a prokaryotic cell. Accordingly, both prokaryotic and eukaryotic cells are included, e.g., bacteria, fungi, yeast, and the like, especially preferred are cells from yeasts, for example, S. *cerevisiae, Y. lipolytica* and *K. lactis.* Host cells also include, but are not limited to, mammalian cell lines such as CHO, VERO, BHK, HeLa, COS, MDCK, 293, 3T3, WI38, and choroid plexus cell lines.

A recombinant host of the disclosure may comprise any polypeptide as described herein. A recombinant host of the disclosure is capable of producing a steviol glycoside. For example, a recombinant host of the disclosure may be capable of producing one or more of, for example, steviol-13-monoside, steviol-19-monoside, 13-[(β-D-Glucopyranosyl)oxy)kaur-16-en-18-oic acid 2-O-β-D-glucopyranosyl-β-D-glucopyranosyl ester, rubusoside, stevioside, steviol-19-diside, steviolbioside, rebaudioside A, rebaudioside E, rebaudioside D or rebaudioside M.

A recombinant host of the disclosure may comprise one or more recombinant nucleic acid sequences encoding one or more polypeptides having UDP-glycosyltransferase (UGT) activity.

For the purposes of this disclosure, a polypeptide having UGT activity is one which has glycosyltransferase activity (EC 2.4), i.e. that can act as a catalyst for the transfer of a monosaccharide unit from an activated nucleotide sugar (also known as the "glycosyl donor") to a glycosyl acceptor molecule, usually an alcohol. The glycosyl donor for a UGT is typically the nucleotide sugar uridine diphosphate glucose (uracil-diphosphate glucose, UDP-glucose).

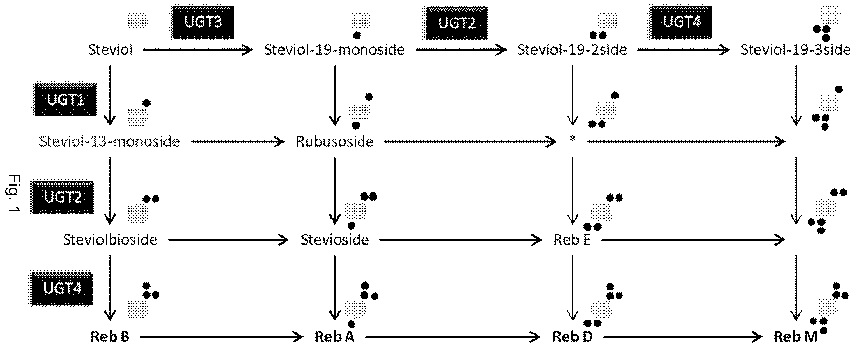

Such additional UGTs may be selected so as to produce a desired steviol glycoside. Schematic diagrams of steviol glycoside formation are set out in Humphrey et al., Plant Molecular Biology (2006) 61: 47-62 and Mohamed et al., J. Plant Physiology 168 (2011) 1136-1141. In addition, Figure 1 sets out a schematic diagram of steviol glycoside formation.

A recombinant host of the disclosure may thus comprise one or more recombinant nucleic acid sequences encoding one or more of:
(i) a polypeptide having UGT74G1 activity;
(ii) a polypeptide having UGT2 activity;
(ii) a polypeptide having UGT85C2 activity; and
(iii) a polypeptide having UGT76G1 activity.

A recombinant yeast suitable for use in the disclosure may comprise a nucleotide sequence encoding a polypeptide capable of catalyzing the addition of a C-13-glucose to steviol. That is to say, a recombinant yeast suitable for use in a method of the disclosure may comprise a UGT which is capable of catalyzing a reaction in which steviol is converted to steviolmonoside.

Such a recombinant yeast suitable for use in a method of the disclosure may comprise a nucleotide sequence encoding a polypeptide having the activity shown by UDP-glycosyltransferase (UGT) UGT85C2, whereby the nucleotide sequence upon transformation of the yeast confers on that yeast the ability to convert steviol to steviolmonoside.

UGT85C2 activity is transfer of a glucose unit to the 13-OH of steviol. Thus, a suitable UGT85C2 may function as a uridine 5'-diphospho glucosyl: steviol 13-OH transferase, and a uridine 5'-diphospho glucosyl: steviol- 19-O-glucoside 13-OH transferase. A functional UGT85C2 polypeptides may also catalyze glucosyl transferase reactions that utilize steviol glycoside substrates other than steviol and steviol- 19-O-glucoside. Such sequences may be referred to as UGT1 sequences herein.

A recombinant yeast suitable for use in the disclosure may comprise a nucleotide sequence encoding a polypeptide which has UGT2 activity.

A polypeptide having UGT2 activity is one which functions as a uridine 5'-diphospho glucosyl: steviol- 13-O-glucoside transferase (also referred to as a steviol-13-monoglucoside 1,2-glucosylase), transferring a glucose moiety to the C-2' of the 13-O-glucose of the acceptor molecule, steviol- 13-O-glucoside. Typically, a suitable UGT2 polypeptide also functions as a uridine 5'-diphospho glucosyl: rubusoside transferase transferring a glucose moiety to the C-2' of the 13-O-glucose of the acceptor molecule, rubusoside.

A polypeptide having UGT2 activity may also catalyze reactions that utilize steviol glycoside substrates other than steviol- 13-O-glucoside and rubusoside, e.g., functional UGT2 polypeptides may utilize stevioside as a substrate, transferring a glucose moiety to the C-2' of the 19-O-glucose residue to produce rebaudioside E. A functional UGT2 polypeptides may also utilize rebaudioside A as a substrate, transferring a glucose moiety to the C-2' of the 19-O-glucose residue to produce rebaudioside D. However, a functional UGT2 polypeptide typically does not transfer a glucose moiety to steviol compounds having a 1,3-bound glucose at the C-13 position, i.e., transfer of a glucose moiety to steviol 1,3-bioside and 1,3-stevioside typically does not occur.

A polypeptide having UGT2 activity may also transfer sugar moieties from donors other than uridine diphosphate glucose. For example, a polypeptide having UGT2 activity act as a uridine 5'-diphospho D-xylosyl: steviol-13-O-glucoside transferase, transferring a xylose moiety to the C-2' of the 13-O-glucose of the acceptor molecule, steviol-13-O-glucoside. As another example, a polypeptide having UGT2 activity may act as a uridine 5'-diphospho L-rhamnosyl: steviol- 13-O-glucoside transferase, transferring a rhamnose moiety to the C-2' of the 13-O-glucose of the acceptor molecule, steviol.

A recombinant yeast suitable for use in the method of the disclosure may comprise a nucleotide sequence encoding a polypeptide having UGT activity may comprise a nucleotide sequence encoding a polypeptide capable of catalyzing the addition of a C-19-glucose to steviolbioside. That is to say, a recombinant yeast of the disclosure may comprise a UGT which is capable of catalyzing a reaction in which steviolbioside is converted to stevioside. Accordingly, such a recombinant yeast may be capable of converting steviolbioside to stevioside. Expression of such a nucleotide sequence may confer on the recombinant yeast the ability to produce at least stevioside.

A recombinant yeast suitable for use in a method of the disclosure may thus also comprise a nucleotide sequence encoding a polypeptide having the activity shown by UDP-glycosyltransferase (UGT) UGT74G1, whereby the nucleotide sequence upon transformation of the yeast confers on the cell the ability to convert steviolbioside to stevioside.

Suitable UGT74G1 polypeptides may be capable of transferring a glucose unit to the 13-OH or the 19-COOH of steviol. A suitable UGT74G1 polypeptide may function as a uridine 5'-diphospho glucosyl: steviol 19-COOH transferase and a uridine 5'-diphospho glucosyl: steviol-13-O-glucoside 19-COOH transferase. Functional UGT74G1 polypeptides also may catalyze glycosyl transferase reactions that utilize steviol glycoside substrates other than steviol and steviol-13-O-glucoside, or that transfer sugar moieties from donors other than uridine diphosphate glucose. Such sequences may be referred to herein as UGT3 sequences.

A recombinant yeast suitable for use in a method the disclosure may comprise a nucleotide sequence encoding a polypeptide capable of catalyzing glucosylation of the C-3' of the glucose at the C-13 position of stevioside. That is to say, a recombinant yeast suitable for use in a method of the disclosure may comprise a UGT which is capable of catalyzing a reaction in which stevioside is converted to rebaudioside A. Accordingly, such a recombinant yeast may be capable of converting stevioside to rebaudioside A. Expression of such a nucleotide sequence may confer on the yeast the ability to produce at least rebaudioside A.

A recombinant yeast suitable for use in a method of the disclosure may thus also comprise a nucleotide sequence encoding a polypeptide having the activity shown by UDP-glycosyltransferase (UGT) UGT76G1, whereby the nucleotide sequence upon transformation of a yeast confers on that yeast the ability to convert stevioside to rebaudioside A.

A suitable UGT76G1 adds a glucose moiety to the C-3'of the C-13-O-glucose of the acceptor molecule, a steviol 1,2 glycoside. Thus, UGT76G1 functions, for example, as a uridine 5'-diphospho glucosyl: steviol 13-O-1,2 glucoside C-3 ' glucosyl transferase and a uridine 5'-diphospho glucosyl: steviol- 19-O-glucose, 13-O-1,2 bioside C-3' glucosyl transferase. Functional UGT76G1 polypeptides may also catalyze glucosyl transferase reactions that utilize steviol glycoside substrates that contain sugars other than glucose, e.g., steviol rhamnosides and steviol xylosides. Such sequences may be referred to herein as UGT4 sequences. A UGT4 may alternatively or in addition be capable of converting RebD to RebM.

A recombinant yeast suitable for use in a method of the disclosure typically comprises nucleotide sequences encoding at least one polypeptide having UGT1 activity, at least one polypeptide having UGT2 activity, at least one polypeptide having UGT3 activity and at least one polypeptide having UGT4 activity. One or more of these nucleic acid sequences may be recombinant. A given nucleic acid may encode a polypeptide having one or more of the above activities. For example, a nucleic acid may encode a polypeptide which has two, three or four of the activities set out above. Preferably, a recombinant yeast for use in the method of the disclosure comprises UGT1, UGT2 and UGT3 and UGT4 activity. Suitable UGT1, UGT2, UGT3 and UGT4 sequences are described in Table 1 of WO2015/007748.

A recombinant host of the disclosure may comprise two or more nucleic acid sequences encoding a polypeptide having any one UGT activity, for example UGT1, 2, 3 or 4, activity. Where a recombinant host of the disclosure comprises two or more nucleic acid sequence encoding a polypeptide having any one UGT activity, those nucleic acid sequences may be the same or different and/or may encode the same or different polypeptides. In particular, a recombinant host of the disclosure may comprise a nucleic acid sequence encoding a two different UGT2 polypeptides.

A recombinant host according to the disclosure may comprise one or more recombinant nucleotide sequence(s) encoding one of more of:
a polypeptide having ent-copalyl pyrophosphate synthase activity;
a polypeptide having ent-Kaurene synthase activity; and
a polypeptide having ent-Kaurene oxidase activity.

A recombinant host according to the disclosure may comprise a recombinant nucleotide sequence encoding a polypeptide having kaurenoic acid 13-hydroxylase activity, other than a KAH polypeptide of the disclosure. That is to say, a recombinant host of the disclosure may comprise a nucleotide sequence or sequences comprising two or more different polypeptides having kaurenoic acid 13-hydroxylase activity one being a KAH polypeptide of the disclosure.

For the purposes of this disclosure, a polypeptide having *ent-copalyl* pyrophosphate synthase (EC 5.5.1.13) is capable of catalyzing the chemical reaction:

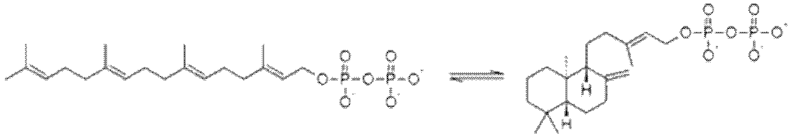

This enzyme has one substrate, geranylgeranyl pyrophosphate, and one product, *ent-*copalyl pyrophosphate. This enzyme participates in gibberellin biosynthesis. This enzyme belongs to the family of isomerase, specifically the class of intramolecular lyases. The systematic name of this enzyme class is ent-copalyl-diphosphate lyase (decyclizing). Other names in common use include having ent-copalyl pyrophosphate synthase, ent-kaurene synthase A, and ent-kaurene synthetase A.

Suitable nucleic acid sequences encoding an ent-copalyl pyrophosphate synthase may for instance comprise a sequence as set out in SEQ ID. NO: 1, 3, 5, 7, 17, 19, 59, 61, 141, 142, 151, 152, 153, 154, 159, 160, 182 or 184 of WO2015/007748.

For the purposes of this disclosure, a polypeptide having ent-kaurene synthase activity (EC 4.2.3.19) is a polypeptide that is capable of catalyzing the chemical reaction:
ent-copalyl diphosphate 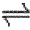 ent-kaurene + diphosphate

Hence, this enzyme has one substrate, ent-copalyl diphosphate, and two products, ent-kaurene and diphosphate.

This enzyme belongs to the family of lyases, specifically those carbon-oxygen lyases acting on phosphates. The systematic name of this enzyme class is ent-copalyl-diphosphate diphosphate-lyase (cyclizing, ent-kaurene-forming). Other names in common use include ent-kaurene synthase B, ent-kaurene synthetase B, ent-copalyl-diphosphate diphosphate-lyase, and (cyclizing). This enzyme participates in diterpenoid biosynthesis.

Suitable nucleic acid sequences encoding an ent-Kaurene synthase may for instance comprise a sequence as set out in SEQ ID. NO: 9, 11, 13, 15, 17, 19, 63, 65, 143, 144, 155, 156, 157, 158, 159, 160, 183 or 184 of WO2015/007748.

ent-copalyl diphosphate synthases may also have a distinct ent-kaurene synthase activity associated with the same protein molecule. The reaction catalyzed by ent-kaurene synthase is the next step in the biosynthetic pathway to gibberellins. The two types of enzymic activity are distinct, and site-directed mutagenesis to suppress the ent-kaurene synthase activity of the protein leads to build up of ent-copalyl pyrophosphate.

Accordingly, a single nucleotide sequence used in a recombinant host of the disclosure may encode a polypeptide having ent-copalyl pyrophosphate synthase activity and ent-kaurene synthase activity. Alternatively, the two activities may be encoded two distinct, separate nucleotide sequences.

For the purposes of this disclosure, a polypeptide having ent-kaurene oxidase activity (EC 1.14.13.78) is a polypeptide which is capable of catalysing three successive oxidations of the 4-methyl group of ent-kaurene to give kaurenoic acid. Such activity typically requires the presence of a cytochrome P450.

Suitable nucleic acid sequences encoding an ent-Kaurene oxidase may for instance comprise a sequence as set out in SEQ ID. NO: 21, 23, 25, 67, 85, 145, 161, 162, 163, 180 or 186 of WO2015/007748.

Suitable nucleic acid sequences encoding a kaurenoic acid 13-hydroxylase, other than a KAH polypeptide of the disclosure, may for instance comprise a sequence as set out in SEQ ID. NO: 27, 29, 31, 33, 69, 89, 91, 93, 95, 97, 146, 164, 165, 166, 167 or 185 of WO2015/007748.

A recombinant host of the disclosure may comprise a recombinant nucleic acid sequence encoding a polypeptide having NADPH-cytochrome p450 reductase activity. That is to say, a recombinant host of the disclosure may be capable of expressing a nucleotide sequence encoding a polypeptide having NADPH-cytochrome p450 reductase activity. For the purposes of the disclosure, a polypeptide having NADPH-Cytochrome P450 reductase activity (EC 1.6.2.4; also known as NADPH:ferrihemoprotein oxidoreductase, NADPH:hemoprotein oxidoreductase, NADPH:P450 oxidoreductase, P450 reductase, POR, CPR, CYPOR) is typically one which is a membrane-bound enzyme allowing electron transfer to cytochrome P450 in the microsome of the host cell from a FAD- and FMN-containing enzyme NADPH:cytochrome P450 reductase (POR; EC 1.6.2.4).

In a recombinant host of the disclosure, the ability of the host to produce geranylgeranyl diphosphate (GGPP) may be upregulated. Upregulated in the context of this disclosure implies that the recombinant host produces more GGPP than an equivalent non-recombinant host.

Accordingly, a recombinant host of the disclosure may comprise one or more nucleotide sequence(s) encoding hydroxymethylglutaryl-CoA reductase, farnesyl-pyrophosphate synthetase and geranylgeranyl diphosphate synthase, whereby the nucleotide sequence(s) upon transformation of the microorganism confer(s) on the microorganism the ability to produce elevated levels of GGPP. Thus, a recombinant host according to the disclosure may comprise one or more recombinant nucleic acid sequence(s) encoding one or more of hydroxymethylglutaryl-CoA reductase, farnesyl-pyrophosphate synthetase and geranylgeranyl diphosphate synthase.

Accordingly, a recombinant host of the disclosure may comprise nucleic acid sequences encoding one or more of:
a polypeptide having hydroxymethylglutaryl-CoA reductase activity;
a polypeptide having farnesyl-pyrophosphate synthetase activity;
a polypeptide having geranylgeranyl diphosphate synthase activity.

A host or host cell as defined herein is an organism suitable for genetic manipulation and one which may be cultured at cell densities useful for industrial production of a target product. A suitable host may be a microorganism, for example one which may be maintained in a fermentation device. A host cell may be a host cell found in nature or a host cell derived from a parent host cell after genetic manipulation or classical mutagenesis.

As used herein, a recombinant host is one which is genetically modified or transformed/transfected with one or more of the nucleotide sequences as defined herein. The presence of the one or more such nucleotide sequences alters the ability of the microorganism to produce steviol or a steviol glycoside, in particular one or more steviol glycosides. A non-recombinant host, i.e. one that is not transformed/transfected or genetically modified, typically does not comprise one or more of the nucleotide sequences enabling the cell to produce a steviol glycoside. Hence, a non-recombinant host is typically a host that does not naturally produce a steviol glycoside, although a host which naturally produces a steviol or a steviol glycoside and which has been modified according to the disclosure (and which thus has an altered ability to produce a steviol glycoside) is considered a recombinant host according to the disclosure.

In particular, it may be possible that the enzymes selected from the group consisting of ent-copalyl pyrophosphate synthase, ent-Kaurene synthase, ent-Kaurene oxidase, and kaurenoic acid 13-hydroxylase, UGTs, hydroxymethylglutaryl-CoA reductase, farnesyl-pyrophosphate synthetase, geranylgeranyl diphosphate synthase and NADPH-cytochrome p450 reductase are native to the host and that transformation with one or more of the nucleotide sequences encoding these enzymes may not be required to confer the host cell the ability to produce steviol or a steviol glycoside. A preferred host according to the present disclosure may be a recombinant host which is naturally capable of producing GGPP (i.e. in its non-recombinant form).

Further improvement of steviol or steviol glycoside production by the host microorganism may be obtained by classical strain improvement.

A host cell may be a prokaryotic, archaebacterial or eukaryotic host cell.

A prokaryotic host cell may be, but is not limited to, a bacterial host cell. A eukaryotic host cell may be, but is not limited to, a yeast, a fungus, an amoeba, an algae, an animal, an insect host cell.

A eukaryotic host cell may be a fungal host cell. "Fungi" include all species of the subdivision *Eumycotina* (Alexopoulos, C. J., 1962, In: Introductory Mycology, John Wiley & Sons, Inc., New York). The term fungus thus includes among others filamentous fungi and yeast.

"Filamentous fungi" are herein defined as eukaryotic microorganisms that include all filamentous forms of the subdivision *Eumycotina* and *Oomycota* (as defined by Hawksworth *et al.,* 1995, supra). The filamentous fungi are characterized by a mycelial wall composed of chitin, cellulose, glucan, chitosan, mannan, and other complex polysaccharides. Vegetative growth is by hyphal elongation and carbon catabolism is obligatory aerobic. Filamentous fungal strains include, but are not limited to, strains of *Acremonium, Aspergillus, Agaricus, Aureobasidium, Cryptococcus, Corynascus, Chrysosporium, Filibasidium, Fusarium, Humicola, Magnaporthe, Monascus, Mucor, Myceliophthora, Mortierella, Neocallimastix, Neurospora, Paecilomyces, Penicillium, Piromyces, Phanerochaete Podospora, Pycnoporus, Rhizopus, Schizophyllum, Sordaria, Talaromyces, Rasmsonia, Thermoascus, Thielavia, Tolypocladium, Trametes* and *Trichoderma.* Preferred filamentous fungal strains that may serve as host cells belong to the species *Aspergillus niger, Aspergillus oryzae, Aspergillus fumigatus, Penicillium chrysogenum, Penicillium citrinum, Acremonium chrysogenum, Trichoderma reesei, Rasamsonia emersonii* (formerly known as *Talaromyces emersonii*)*, Aspergillus sojae, Chrysosporium lucknowense, Myceliophtora thermophyla.* Reference host cells for the comparison of fermentation characteristics of transformed and untransformed cells, include e.g. *Aspergillus niger* CBS120.49, CBS 513.88, *Aspergillus oryzae* ATCC16868, ATCC 20423, IFO 4177, ATCC 1011, ATCC 9576, ATCC14488-14491, ATCC 11601, ATCC12892, *Aspergillus fumigatus* AF293 (CBS101355), P. *chrysogenum* CBS 455.95, *Penicillium citrinum* ATCC 38065, *Penicillium chrysogenum* P2, *Acremonium chrysogenum* ATCC 36225, ATCC 48272, *Trichoderma reesei* ATCC 26921, ATCC 56765, ATCC 26921, *Aspergillus sojae* ATCC11906, *Chrysosporium lucknowense* ATCC44006 and derivatives of all of these strains. Particularly preferred as filamentous fungal host cell are *Aspergillus niger* CBS 513.88 and derivatives thereof.

A eukaryotic host cell may be a yeast cell. Preferred yeast host cells may be selected from the genera: *Saccharomyces* (e.g., *S. cerevisiae, S. bayanus, S. pastorianus, S. carlsbergensis), Brettanomyces, Kluyveromyces, Candida* (e.g., *C. krusei, C. revkaufi, C. pulcherrima, C. tropicalis, C. utilis), Issatchenkia* (eg. *I. orientalis) Pichia* (e.g., *P. pastoris), Schizosaccharomyces, Hansenula, Kloeckera, Pachysolen, Schwanniomyces, Trichosporon, Yarrowia* (e.g., *Y. lipolytica* (formerly classified as *Candida lipolytica)), Yamadazyma.*

Prokaryotic host cells may be bacterial host cells. Bacterial host cell may be Gram negative or Gram positive bacteria. Examples of bacteria include, but are not limited to, bacteria belonging to the genus *Bacillus* (e.g., B. *subtilis, B. amyloliquefaciens, B. licheniformis, B. puntis, B. megaterium, B. halodurans, B. pumilus,), Acinetobacter, Nocardia, Xanthobacter, Escherichia* (e.g., E. *coli* (e.g., strains DH 1 OB, Stbl2, DH5-alpha, DB3, DB3.1 ), DB4, DB5, JDP682 and ccdA-over (e.g., U.S. application No. 09/518,188))), *Streptomyces, Erwinia, Klebsiella, Serratia* (e.g., *S. marcessans), Pseudomonas* (e.g., P. *aeruginosa), Salmonella* (e.g., *S. typhimurium, S. typhi).* Bacteria also include, but are not limited to, photosynthetic bacteria (e.g., green non-sulfur bacteria (e.g., *Choroflexus* bacteria (e.g., *C. aurantiacus), Chloronema* (e.g., *C. gigateum)),* green sulfur bacteria (e.g., *Chlorobium bacteria* (e.g., *C. limicola), Pelodictyon* (e.g., *P. luteolum),* purple sulfur bacteria (e.g., *Chromatium* (e.g., *C. okenii)),* and purple non-sulfur bacteria (e.g., *Rhodospirillum* (e.g., *R. rubrum), Rhodobacter* (e.g. R. *sphaeroides, R. capsulatus),* and *Rhodomicrobium* bacteria (e.g., R. *vanellii)).*

Host Cells may be host cells from non-microbial organisms. Examples of such cells, include, but are not limited to, insect cells (e.g., *Drosophila* (e.g., *D. melanogaster), Spodoptera* (e.g., S. *frugiperda* Sf9 or Sf21 cells) and *Trichoplusa* (e.g., High-Five cells); nematode cells (e.g., *C. elegans* cells); avian cells; amphibian cells (e.g., *Xenopus laevis* cells); reptilian cells; and mammalian cells (e.g., NIH3T3, 293, CHO, COS, VERO, C127, BHK, Per-C6, Bowes melanoma and HeLa cells).

A recombinant host according to the present disclosure may be able to grow on any suitable carbon source known in the art and convert it to a steviol glycoside, e.g. a steviol glycoside. The recombinant host may be able to convert directly plant biomass, celluloses, hemicelluloses, pectines, rhamnose, galactose, fucose, maltose, maltodextrines, ribose, ribulose, or starch, starch derivatives, sucrose, glucose, lactose or glycerol. Hence, a preferred host expresses enzymes such as cellulases (endocellulases and exocellulases) and hemicellulases (e.g. endo- and exo-xylanases, arabinases) necessary for the conversion of cellulose into glucose monomers and hemicellulose into xylose and arabinose monomers, pectinases able to convert pectines into glucuronic acid and galacturonic acid or amylases to convert starch into glucose monomers. Preferably, the host is able to convert a carbon source selected from the group consisting of glucose, xylose, arabinose, sucrose, lactose and glycerol. The host cell may for instance be a eukaryotic host cell as described in WO03/062430, WO06/009434, EP1499708B1, WO2006096130 or WO04/099381.

Thus, in a further aspect, the disclosure also provides a process for the preparation of a steviol glycoside which comprises fermenting a recombinant host of the disclosure which is capable of producing at least one steviol glycoside in a suitable fermentation medium, and optionally recovering the steviol glycoside.

The steviol glycoside may be, for example, steviol-13-monoside, steviol-19-monoside, 13-[(β-D-Glucopyranosyl)oxy)kaur-16-en-18-oic acid 2-O-β-D-glucopyranosyl-β-D-glucopyranosyl ester, rubusoside, stevioside, steviol-19-diside, steviolbioside, rebA, rebaudioside B, rebaudioside C, rebaudioside E, rebaudioside D or rebaudioside M.

The fermentation medium used in the process for the production of a steviol glycoside may be any suitable fermentation medium which allows growth of a particular host cell. The essential elements of the fermentation medium are known to the person skilled in the art and may be adapted to the host cell selected.

Preferably, the fermentation medium comprises a carbon source selected from the group consisting of plant biomass, celluloses, hemicelluloses, pectines, rhamnose, galactose, fucose, fructose, maltose, maltodextrines, ribose, ribulose, or starch, starch derivatives, glucose, sucrose, lactose, fatty acids, triglycerides and glycerol. Preferably, the fermentation medium also comprises a nitrogen source such as urea, or an ammonium salt such as ammonium sulphate, ammonium chloride, ammonium nitrate or ammonium phosphate.

The fermentation process according to the present disclosure may be carried out in batch, fed-batch or continuous mode. A separate hydrolysis and fermentation (SHF) process or a simultaneous saccharification and fermentation (SSF) process may also be applied. A combination of these fermentation process modes may also be possible for optimal productivity. A SSF process may be particularly attractive if starch, cellulose, hemicelluose or pectin is used as a carbon source in the fermentation process, where it may be necessary to add hydrolytic enzymes, such as cellulases, hemicellulases or pectinases to hydrolyse the substrate.

The recombinant host used in the process for the preparation of a steviol glycoside may be any suitable recombinant host as defined herein above. It may be advantageous to use a recombinant eukaryotic host according to the disclosure in the process since most eukaryotic cells do not require sterile conditions for propagation and are insensitive to bacteriophage infections. In addition, eukaryotic host cells may be grown at low pH to prevent bacterial contamination.

The recombinant host according to the present disclosure may be a facultative anaerobic microorganism. A facultative anaerobic recombinant host can be propagated aerobically to a high cell concentration. This anaerobic phase can then be carried out at high cell density which reduces the fermentation volume required substantially, and may minimize the risk of contamination with aerobic microorganisms.

The fermentation process for the production of a steviol glycoside according to the present disclosure may be an aerobic or an anaerobic fermentation process.

An anaerobic fermentation process may be herein defined as a fermentation process run in the absence of oxygen or in which substantially no oxygen is consumed, preferably less than 5, 2.5 or 1 mmol/L/h, and wherein organic molecules serve as both electron donor and electron acceptors. The fermentation process according to the present disclosure may also first be run under aerobic conditions and subsequently under anaerobic conditions.

The fermentation process may also be run under oxygen-limited, or micro-aerobical, conditions. Alternatively, the fermentation process may first be run under aerobic conditions and subsequently under oxygen-limited conditions. An oxygen-limited fermentation process is a process in which the oxygen consumption is limited by the oxygen transfer from the gas to the liquid. The degree of oxygen limitation is determined by the amount and composition of the ingoing gasflow as well as the actual mixing/mass transfer properties of the fermentation equipment used.

The production of a steviol glycoside in the process according to the present disclosure may occur during the growth phase of the host cell, during the stationary (steady state) phase or during both phases. It may be possible to run the fermentation process at different temperatures.

The process for the production of a steviol glycoside may be run at a temperature which is optimal for the recombinant host. The optimum growth temperature may differ for each transformed recombinant host and is known to the person skilled in the art. The optimum temperature might be higher than optimal for wild type organisms to grow the organism efficiently under non-sterile conditions under minimal infection sensitivity and lowest cooling cost. Alternatively, the process may be carried out at a temperature which is not optimal for growth of the recombinant host.

The process for the production of a steviol glycoside according to the present disclosure may be carried out at any suitable pH value. If the recombinant host is a yeast, the pH in the fermentation medium preferably has a value of below 6, preferably below 5,5, preferably below 5, preferably below 4,5, preferably below 4, preferably below pH 3,5 or below pH 3,0, or below pH 2,5, preferably above pH 2. An advantage of carrying out the fermentation at these low pH values is that growth of contaminant bacteria in the fermentation medium may be prevented.

Such a process may be carried out on an industrial scale. The product of such a process is one or more steviol glycosides, such one or more of, for example, steviol-13-monoside, steviol-19-monoside, 13-[(β-D-Glucopyranosyl)oxy)kaur-16-en-18-oic acid 2-O-β-D-glucopyranosyl-β-D-glucopyranosyl ester, rubusoside, stevioside, steviol-19-diside, steviolbioside, rebaudioside A, rebaudioside E, rebaudioside D or rebaudioside M.

Recovery of steviol glycoside(s) from the fermentation medium may be performed by known methods in the art, for instance by distillation, vacuum extraction, solvent extraction, or evaporation.

In the process for the production of a steviol glycoside according to the disclosure, it may be possible to achieve a concentration of above 5 mg/l fermentation broth, preferably above 10 mg/l, preferably above 20 mg/l, preferably above 30 mg/l fermentation broth, preferably above 40 mg/l, more preferably above 50 mg/l, preferably above 60 mg/l, preferably above 70, preferably above 80 mg/l, preferably above 100 mg/l, preferably above 1 g/l, preferably above 5 g/l, preferably above 10 g/l, but usually below 70 g/l.

The disclosure further provides a fermentation broth comprising a steviol glycoside and the recombinant host cell according to the disclosure.

In the event that one or more steviol glycosides is expressed within the microorganism, such cells may need to be treated so as to release them. Preferentially, at least one steviol glycoside, for example rebA, reb D or rebM, is produced extracellularly.

A broth according to the disclosure may comprise more than at least one steviol glycoside, such as rebA, rebD or rebM, as compared with a broth produced from a recombinant host in which a reference polypeptide is expressed instead of a polypeptide of the disclosure.

A broth according to the disclosure may comprise less of at least one non-steviol glycoside, for example one or more kaurenoic acid glycosides, as compared with a broth produced from a recombinant host in which a reference polypeptide is expressed instead of a polypeptide of the disclosure.

Disclosed but not claimed is a steviol glycoside obtained by a process according to the disclosure for the preparation of a steviol glycoside or obtainable from a fermentation broth of the disclosure. Such a steviol glycoside may be a non- naturally occurring steviol glycoside, that is to say one which is not produced in plants.

Disclosed but not claimed is a composition comprising one or more, for example two or more, steviol glycosides obtainable by a process of the disclosure for the preparation of a steviol glycoside or obtainable from a fermentation broth of the disclosure. In such a composition, one or more of the steviol glycosides may be a non- naturally occurring steviol glycoside, that is to say one which is not produced in plants.

A steviol glycoside or composition produced by the fermentation process according to the present disclosure may be used in any application known for such compounds. In particular, they may for instance be used as a sweetener, for example in a food or a beverage. According to the disclosure therefore, there is provided a foodstuff, feed or beverage which comprises a steviol glycoside or a composition of the disclosure.

For example a steviol glycoside or a composition may be formulated in soft drinks, as a tabletop sweetener, chewing gum, dairy product such as yoghurt (eg. plain yoghurt), cake, cereal or cereal-based food, nutraceutical, pharmaceutical, edible gel, confectionery product, cosmetic, toothpastes or other oral cavity composition, etc. In addition, a steviol glycoside or a composition of the disclosure can be used as a sweetener not only for drinks, foodstuffs, and other products dedicated for human consumption, but also in animal feed and fodder with improved characteristics.

Disclosed but not claimed is, *inter alia,* a foodstuff, feed or beverage which comprises a steviol glycoside prepared according to a process of the disclosure.

During the manufacturing of foodstuffs, drinks, pharmaceuticals, cosmetics, table top products, chewing gum the conventional methods such as mixing, kneading, dissolution, pickling, permeation, percolation, sprinkling, atomizing, infusing and other methods can be used.

A steviol glycoside or a composition can be used in dry or liquid forms. It can be added before or after heat treatment of food products. The amount of the sweetener depends on the purpose of usage. It can be added alone or in the combination with other compounds.

Compounds produced according to the method of the disclosure may be blended with one or more further non-caloric or caloric sweeteners. Such blending may be used to improve flavour or temporal profile or stability. A wide range of both non-caloric and caloric sweeteners may be suitable for blending with a steviol glycoside or a composition of the disclosure. For example, non-caloric sweeteners such as mogroside, monatin, aspartame, acesulfame salts, cyclamate, sucralose, saccharin salts or erythritol. Caloric sweeteners suitable for blending with a steviol glycoside or a composition of the disclosure include sugar alcohols and carbohydrates such as sucrose, glucose, fructose and HFCS. Sweet tasting amino acids such as glycine, alanine or serine may also be used.

A steviol glycoside or a composition can be used in the combination with a sweetener suppressor, such as a natural sweetener suppressor. It may be combined with an umami taste enhancer, such as an amino acid or a salt thereof.

A steviol glycoside or a composition can be combined with a polyol or sugar alcohol, a carbohydrate, a physiologically active substance or functional ingredient (for example a carotenoid, dietary fiber, fatty acid, saponin, antioxidant, nutraceutical, flavonoid, isothiocyanate, phenol, plant sterol or stanol (phytosterols and phytostanols), a polyols, a prebiotic, a probiotic, a phytoestrogen, soy protein, sulfides/thiols, amino acids, a protein, a vitamin, a mineral, and/or a substance classified based on a health benefits, such as cardiovascular, cholesterol-reducing or anti-inflammatory.

A composition with a steviol glycoside or a composition may include a flavoring agent, an aroma component, a nucleotide, an organic acid, an organic acid salt, an inorganic acid, a bitter compound, a protein or protein hydrolyzate, a surfactant, a flavonoid, an astringent compound, a vitamin, a dietary fiber, an antioxidant, a fatty acid and/or a salt.

A steviol glycoside or a composition may be applied as a high intensity sweetener to produce zero calorie, reduced calorie or diabetic beverages and food products with improved taste characteristics. Also it can be used in drinks, foodstuffs, pharmaceuticals, and other products in which sugar cannot be used.

In addition, a steviol glycoside or a composition may be used as a sweetener not only for drinks, foodstuffs, and other products dedicated for human consumption, but also in animal feed and fodder with improved characteristics.

The examples of products where a steviol glycoside or a composition can be used as a sweetening compound can be as alcoholic beverages such as vodka, wine, beer, liquor, sake, etc.; natural juices, refreshing drinks, carbonated soft drinks, diet drinks, zero calorie drinks, reduced calorie drinks and foods, yogurt drinks, instant juices, instant coffee, powdered types of instant beverages, canned products, syrups, fermented soybean paste, soy sauce, vinegar, dressings, mayonnaise, ketchups, curry, soup, instant bouillon, powdered soy sauce, powdered vinegar, types of biscuits, rice biscuit, crackers, bread, chocolates, caramel, candy, chewing gum, jelly, pudding, preserved fruits and vegetables, fresh cream, jam, marmalade, flower paste, powdered milk, ice cream, sorbet, vegetables and fruits packed in bottles, canned and boiled beans, meat and foods boiled in sweetened sauce, agricultural vegetable food products, seafood, ham, sausage, fish ham, fish sausage, fish paste, deep fried fish products, dried seafood products, frozen food products, preserved seaweed, preserved meat, tobacco, medicinal products, and many others. In principal it can have unlimited applications.

The sweetened composition comprises a beverage, non-limiting examples of which include non-carbonated and carbonated beverages such as colas, ginger ales, root beers, ciders, fruit-flavored soft drinks (e.g., citrus-flavored soft drinks such as lemon-lime or orange), powdered soft drinks, and the like; fruit juices originating in fruits or vegetables, fruit juices including squeezed juices or the like, fruit juices containing fruit particles, fruit beverages, fruit juice beverages, beverages containing fruit juices, beverages with fruit flavorings, vegetable juices, juices containing vegetables, and mixed juices containing fruits and vegetables; sport drinks, energy drinks, near water and the like drinks (e.g., water with natural or synthetic flavorants); tea type or favorite type beverages such as coffee, cocoa, black tea, green tea, oolong tea and the like; beverages containing milk components such as milk beverages, coffee containing milk components, cafe au lait, milk tea, fruit milk beverages, drinkable yogurt, lactic acid bacteria beverages or the like; and dairy products.

Generally, the amount of sweetener present in a sweetened composition varies widely depending on the particular type of sweetened composition and its desired sweetness. Those of ordinary skill in the art can readily discern the appropriate amount of sweetener to put in the sweetened composition.

A steviol glycoside or a composition can be used in dry or liquid forms. It can be added before or after heat treatment of food products. The amount of the sweetener depends on the purpose of usage. It can be added alone or in the combination with other compounds.

During the manufacturing of foodstuffs, drinks, pharmaceuticals, cosmetics, table top products, chewing gum the conventional methods such as mixing, kneading, dissolution, pickling, permeation, percolation, sprinkling, atomizing, infusing and other methods can be used.

Thus, compositions can be made by any method known to those skilled in the art that provide homogenous even or homogeneous mixtures of the ingredients. These methods include dry blending, spray drying, agglomeration, wet granulation, compaction, co-crystallization and the like.

In solid form a steviol glycoside or a composition can be provided to consumers in any form suitable for delivery into the comestible to be sweetened, including sachets, packets, bulk bags or boxes, cubes, tablets, mists, or dissolvable strips. The composition can be delivered as a unit dose or in bulk form.

For liquid sweetener systems and compositions convenient ranges of fluid, semi-fluid, paste and cream forms, appropriate packing using appropriate packing material in any shape or form shall be invented which is convenient to carry or dispense or store or transport any combination containing any of the above sweetener products or combination of product produced above.

The composition may include various bulking agents, functional ingredients, colorants, flavors.

The terms "sequence homology" or "sequence identity" are used interchangeably herein. For the purpose of this disclosure, it is defined here that in order to determine the percentage of sequence homology or sequence identity of two amino acid sequences or of two nucleic acid sequences, the sequences are aligned for optimal comparison purposes. In order to optimize the alignment between the two sequences gaps may be introduced in any of the two sequences that are compared. Such alignment can be carried out over the full length of the sequences being compared. Alternatively, the alignment may be carried out over a shorter length, for example over about 20, about 50, about 100 or more nucleic acids/based or amino acids. The sequence identity is the percentage of identical matches between the two sequences over the reported aligned region.

A comparison of sequences and determination of percentage of sequence identity between two sequences is accomplished using a mathematical algorithm. The skilled person will be aware of the fact that several different computer programs are available to align two sequences and determine the identity between two sequences (Kruskal, J. B. (1983) An overview of sequence comparison In D. Sankoff and J. B. Kruskal, (ed.), Time warps, string edits and macromolecules: the theory and practice of sequence comparison, pp. 1-44 Addison Wesley). The percent sequence identity between two amino acid sequences or between two nucleotide sequences is determined using the Needleman and Wunsch algorithm for the alignment of two sequences. (Needleman, S. B. and Wunsch, C. D. (1970) J. Mol. Biol. 48, 443-453). Both amino acid sequences and nucleotide sequences can be aligned by the algorithm. The Needleman-Wunsch algorithm has been implemented in the computer program NEEDLE. For the purpose of this disclosure the NEEDLE program from the EMBOSS package was used (version 2.8.0 or higher, EMBOSS: The European Molecular Biology Open Software Suite (2000) Rice,P. Longden,I. and Bleasby, A. Trends in Genetics 16, (6) pp 276-277, http://emboss.bioinformatics.nl/). For protein sequences EBLOSUM62 is used for the substitution matrix. For nucleotide sequence, EDNAFULL is used. The parameters used are a gap-open penalty of 10 and a gap extension penalty of 0.5.

After alignment by the program NEEDLE as described above the percentage of sequence identity between a query sequence and a sequence of the disclosure is calculated as follows: Number of corresponding positions in the alignment showing an identical amino acid or identical nucleotide in both sequences divided by the total length of the alignment after subtraction of the total number of gaps in the alignment. The identity defined as herein can be obtained from NEEDLE by using the NOBRIEF option and is labelled in the output of the program as "longest-identity".

The nucleic acid and protein sequences of the present disclosure can further be used as a "query sequence" to perform a search against public databases to, for example, identify other family members or related sequences. Such searches can be performed using the NBLAST and XBLAST programs (version 2.0) of Altschul, et al. (1990) J. Mol. Biol. 215:403-10. BLAST nucleotide searches can be performed with the NBLAST program, score = 100, word length = 12 to obtain nucleotide sequences homologous to nucleic acid molecules of the disclosure. BLAST protein searches can be performed with the XBLAST program, score = 50, word length = 3 to obtain amino acid sequences homologous to protein molecules of the disclosure. To obtain gapped alignments for comparison purposes, Gapped BLAST can be utilized as described in Altschul et al., (1997) Nucleic Acids Res. 25(17): 3389-3402. When utilizing BLAST and Gapped BLAST programs, the default parameters of the respective programs (e.g., XBLAST and NBLAST) can be used. See the homepage of the National Center for Biotechnology Information at http://www.ncbi.nlm.nih.gov/.

A reference herein to a patent document or other matter which is given as prior art is not to be taken as an admission that that document or matter was known or that the information it contains was part of the common general knowledge as at the priority date of any of the claims.

The present disclosure is further illustrated by the following Examples:

### EXAMPLES

### General

Standard genetic techniques, such as overexpression of enzymes in the host cells, as well as for additional genetic modification of host cells, are known methods in the art, such as described in Sambrook and Russel (2001) "Molecular Cloning: A Laboratory Manual (3rd edition), Cold Spring Harbor Laboratory, Cold Spring Harbor Laboratory Press*,* or F. Ausubel et al, eds., "Current protocols in molecular biology", Green Publishing and Wiley Interscience, New York (1987). Methods for transformation and genetic modification of fungal host cells are known from e.g. EP-A-0 635 574, WO 98/46772, WO 99/60102 and WO 00/37671.

### Example 1. KAH expression in Yarrowia lipolytica

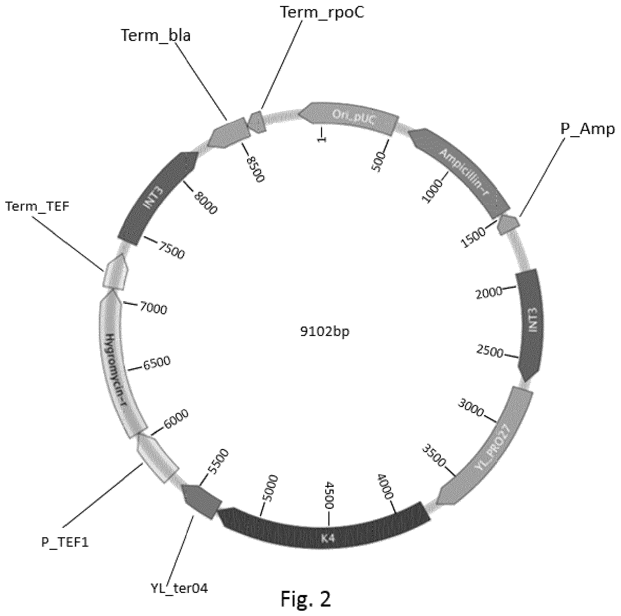

Different kaurenoic acid hydroxylase (KAH)-encoding genes (see Table 1 below) were ordered as cloned genes in a vector at DNA2.0, and contained the INT3 integration flanks (which allow homologous recombination in *Y. lipolytica),* and promotor-orf-terminator for KAH and HygB (encoding for resistance against hygromycin). See Fig. 2 for the plasmid map.

**Table 1. KAH genes**

| **Name** | **Amino acid sequence** | **Nucleic acid sequence** | **Substitutions in comparison with SEQ ID NO: 1** |
|---|---|---|---|
| KAH4_m4 | SEQ ID NO: 3 | SEQ ID NO: 4 | |
| KAH4_p18 | SEQ ID NO: 5 | SEQ ID NO: 6 | I136M |
| KAH4_p19 | SEQ ID NO: 7 | SEQ ID NO: 8 | F336S |
| KAH4_p20 | SEQ ID NO: 9 | SEQ ID NO: 10 | I136V |
| KAH4_p21 | SEQ ID NO: 11 | SEQ ID NO: 12 | S248N |
| KAH4_p22 | SEQ ID NO: 13 | SEQ ID NO: 14 | I403G |

The expression pathways containing integration flanks, KAH and HygB expression cassettes were PCR-amplified from the plasmids. The purified PCR products were transformed to *Y. lipolytica* strain STV2226, and hygromycin resistant colonies were selected. The STV2226 strain already expresses all the genes that are required for steviol glycosides production to produce steviol glycosides, except for KAH. The gene content of this strain is given below in Table 2. Construction of similar strains has been described in more detail in patent application numbers WO2013/110673 and WO2015/007748. The STV2226 strain contains an internal deletion of 1658 bp in the ku70 gene, to increase the efficiency of targeted integration.

**Table 2. Genotype of strain STV2226. Between brackets indicates the gene copy number present in the strain**

| **Strain name** | **Genotype** |
|---|---|
| STV2226 | MATB ku70Δ tHMG (2; SEQ ID NO: 15) GGS (2; SEQ ID NO: 16) CarG (1; SEQ ID NO: 17) CPS (5; SEQ ID NO: 18) KS (4; SEQ ID NO: 19) KO (2; SEQ ID NO: 20) CPR3 (2; SEQ ID NO: 21) UGT1 (3; SEQ ID NO: 22) UGT2 (2; SEQ ID NO: 23) UGT3 (2; SEQ ID NO: 24) UGT4 (3; SEQ ID NO: 25) |

### Example 2. Production of glycosylated kaurenoic acid and steviol glycosides in strains expressing KAH genes

STV2226 transformed with the different KAH genes were plated on YPhD plates containing hygromycin, single colony isolates were obtained, and a production test was performed: as pre-culture 200 µl YEP with glucose was inoculated with colony material from YEPh-D agar plates containing hygromycin. The pre-culture was incubated 72 hours in an Infors incubator at 30°C, 750 rpm and 80% humidity. 40 µl of pre-culture was used to inoculate 2.5 ml mineral medium with glucose as carbon source. These production cultures were incubated 120 hours in an Infors incubator at 30°C, 550 rpm, 80% humidity. The production cultures were pelleted by centrifugation at 3000g for 10 minutes. After centrifugation supernatant was transferred and diluted in 33% acetonitrile and analyzed for steviol, steviol glycosides, kaurenoic acid (KA) and glycosylated kaurenoic acid (KA-glycosides) using LC/MS. To represent the data, steviol glycosides titers (mM) and KA-glycoside titers (mM) were normalized to the titers obtained with STV2226 transformed with KAH4_m4 (SEQ ID NO: 2). For an overview of the results, see Table 3.

**Table 3. Production of KA-glycosides and steviol-glycosides. Values represent averages of around nine replicates for most genes, and at least of two replicates for all genes. Steviol glycosides (mM) and KA-glycosides (mM) were normalized to the production in strain STV2226 transformed with KAH4_m4.**

| **Strain** | **Sum steviol glycosides** | **Sum KA-glycosides** | **Steviol glycosides / KA-glycosides** |
|---|---|---|---|
| STV2226 + KAH4_m4 | 100 | 100 | 4.3 |
| STV2226 + KAH4_p18 | 100 | 47 | 9.2 |
| STV2226 + KAH4_p19 | 154 | 101 | 6.6 |
| STV2226 + KAH4_p20 | 115 | 56 | 8.9 |
| STV2226 + KAH4_p21 | 130 | 64 | 8.9 |
| STV2226 + KAH4_p22 | 106 | 75 | 6.3 |

The sum of steviol glycosides includes steviol-13-monoside, steviol-19-monoside, steviol bioside, rubusoside, Rebaudioside B, stevioside, Rebaudioside A, Rebaudioside E, Rebaudioside D and Rebaudioside M. Sum KA-glycosides includes KA, KA-19-monoglucoside, KA-19-diglucoside and KA-19-triglucoside.

The strains that expressed the KAH genes KAH4_p19, KAH4_p20, KAH4_p21 and KAH4_p22, produced higher titers of steviol glycosides. Expression of some of these genes resulted in 30% or more improvement in total steviol glycosides production compared to KAH4_m4. The formation of the undesired KA-glycosides was greatly reduced for genes KAH4_p18, KAH4_p20, KAH4_p21 and KAH4_p22. The ratio of desired product (steviol glycosides) over undesired byproducts (KA-glycosides) increased for all strains expressing genes KAH4_p18 to KAH4_p22 compared to the strain expressing KAH4_m4, for some KAH genes even by a factor of more than 2. These results illustrate that KAH4_p18 to KAH4_p22 enzymes are beneficial for the production of steviol glycosides.

### Example 3. Production of glycosylated kaurenoic acid and steviol glycosides in bioreactors

Three of the strains, expressing KAH4_m4 (reference), KAH4_p20 and KAH4_p21 constructed as described above were cultivated in 500 mL shake-flasks with 50 ml mineral medium for 3 days at 30°C and 280 rpm. Subsequently, 6ml of the content of the shake-flask was transferred into a fermenter with a starting volume of 0.3 L. The pH was controlled at 5.0 by addition of ammonia (12.5 wt%). Temperature was controlled at 30°C. Glucose concentration was kept limited by controlled glucose feed to the fermenter. The mineral medium of the shake flask and fermentation was based on Verduyn *et al.* (Verduyn C, Postma E, Scheffers WA, Van Dijken JP. Yeast, 1992 Jul;8(7):501-517). Broth samples were diluted in water and 33% acetonitrile and analyzed with LC/MS.

**Table 4. Steviol glycoside and KA-glycoside production in bioreactors. Steviol glycosides (mM) and KA-glycosides (mM) were normalized to the production in strain STV2226 transformed with KAH4_m4.**

| **Strain** | **Sum steviol glycosides (%)** | **Sum KA-glycosides (%)** | **Sum steviol glycosides / sum KA-glycosides** |
|---|---|---|---|
| STV2226 + KAH4_m4 | 100 | 100 | 11 |
| STV2226 + KAH4_p20 | 120 | 63 | 20 |
| STV2226 + KAH4_p21 | 101 | 37 | 29 |

The sum of steviol glycosides includes steviol, steviol-13-monoside, steviol-19-monoside, steviol bioside, rubusoside, Rebaudioside B, stevioside, Rebaudioside A, Rebaudioside E, Rebaudioside D and Rebaudioside M. Sum KA-glycosides includes KA, KA-19-monoglucoside, KA-19-diglucoside and KA-19-triglucoside.

We observed that the amount of steviol glycosides increases when the KAH4_p20 is expressed, resulting in 20% higher production of total steviol glycosides. In addition the amount of KA-glycosides decreases, with more than 35%. When KAH4_p21 is expressed the reduction in KA-glycosides is even higher, with more than 60% reduction. As a result, with KAH4_p20 or with KAH4_p21 expression, the ratio of steviol glycosides over KA-glycosides is increased dramatically compared to the strain expressing KAH4_m4. Use of KAH4_p20 or KAH4_p21 will have a positive effect on the production and purification of steviol glycosides.

## Claims

1. A polypeptide having kaurenoic acid 13-hydroxylase (KAH) activity, which polypeptide comprises an amino acid sequence which, when aligned with a kaurenoic acid 13-hydroxylase comprising the sequence set out in SEQ ID NO: 1 or SEQ ID NO: 3, comprises at least one substitution of an amino acid corresponding to any of amino acids at positions
136, 248, 336, or 403, wherein the substitution to the amino acid corresponding to position 136 is a methionine, alanine, phenylalanine, tryptophan, glutamine, histidine or threonine,
said positions being defined with reference to SEQ ID NO: 1 or SEQ ID NO: 3 and wherein the polypeptide has a modified KAH activity as compared with the reference polypeptide having kaurenoic acid 13-hydroxylase activity according to SEQ ID NO: 3, wherein the modified activity leads to an increase of the ratio between the sum of steviol glycosides and the sum of kaurenoic acid-glycosides when the polypeptide with modified activity is overexpressed in a host cell as compared to the production level of an equivalent host cell which overexpresses the reference polypeptide, wherein the sum of steviol glycosides is the sum of the production level of steviol-13-monoside, steviol-19-monoside, steviol bioside, rubusoside, Rebaudioside B, stevioside, Rebaudioside A, Rebaudioside E, Rebaudioside D and Rebaudioside M, wherein the sum of KA-glycosides is the sum of the production level of KA, KA-19-monoglucoside, KA-19-diglucoside and KA-19-triglucoside, wherein the positions in the polypeptide having kaurenoic acid 13-hydroxylase activity corresponding to any amino acids at position 136, 248, 336 or 403, are identified by aligning the amino acid sequence of the polypeptide with kaurenoic acid 13-hydroxylase activity with the amino acid sequence set out in SEQ ID NO: 1 or SEQ ID NO: 3 using the EMBOSS Needle alignment method, using EBLOSUM62 as a substitution matrix, with a gap-open penalty of 10 and a gap extension penalty of 0.5. and wherein the polypeptide comprises an amino acid sequence having at least 90%, at least 95%, at least 97%, at least 98% or at least 99% sequence identity with SEQ ID NO: 1 or SEQ ID NO: 3.

2. A polypeptide according to claim 1, wherein:
(i) a methionine is present at position 136;
(ii) an asparagine is present at position 248;
(iii) a serine is present at position 336; and/or
(iv) a glycine is present at position 403,
said positions being defined with reference to SEQ ID NO: 1 or SEQ ID NO: 3.

3. A polypeptide according to any one of the preceding claims having kaurenoic acid 13-hydroxylase activity comprising an amino acid sequence having at least about 95% sequence identity, at least 96%, at least 97%, at least 98% or at least 99% sequence identity to any one of SEQ ID NO: 5, 7, 9, 11 or 13.

4. A nucleic acid comprising a sequence encoding a polypeptide according to any one of the preceding claims.

5. A recombinant host cell comprising a nucleic acid according to claim 4, optionally which is capable of producing steviol or a steviol glycoside.

6. A recombinant host cell according to claim 5 which comprises one or more recombinant nucleotide sequence(s) encoding:
a polypeptide having ent-copalyl pyrophosphate synthase activity;
a polypeptide having ent-Kaurene synthase activity; and
a polypeptide having ent-Kaurene oxidase activity; and, optionally,
a polypeptide having kaurenoic acid 13-hydroxylase activity which is different from a polypeptide according to any one of claims 1 to 3.

7. A recombinant host cell according to claim 5 or 6, which comprises a recombinant nucleic acid sequence encoding a polypeptide having NADPH-cytochrome p450 reductase activity.

8. A recombinant host cell according to any one of claims 5 to 7 which comprises a recombinant nucleic acid sequence encoding one or more of:
(i) a polypeptide having UGT74G1 activity;
(ii) a polypeptide having UGT2 activity;
(iii) a polypeptide having UGT85C2 activity; and
(iv) a polypeptide having UGT76G1 activity.

9. A recombinant host cell according to any one of claims 5 to 8, wherein the host cell belongs to one of the genera *Saccharomyces, Aspergillus, Pichia, Kluyveromyces, Candida, Hansenula, Humicola, Issatchenkia, Trichosporon, Brettanomyces, Pachysolen, Yarrowia, Yamadazyma* or *Escherichia,* for example a *Saccharomyces cerevisiae* cell, a *Yarrowia lipolytica* cell, a *Candida krusei* cell, an *Issatchenkia orientalis* cell or an *Escherichia coli* cell.

10. A process for the preparation of steviol or a steviol glycoside which comprises fermenting a recombinant host cell according to any one of claims 5 to 9 in a suitable fermentation medium and, optionally recovering the steviol or steviol glycoside.

11. A fermentation broth comprising a steviol glycoside and the recombinant host cell according to any one of claims 5 to 9.

## Patentansprüche

1. Polypeptid mit Kaurensäure-13-Hydroxylase(KAH)-Aktivität, wobei das Polypeptid eine Aminosäuresequenz umfasst, die bei ihrer Ausrichtung an einer Kaurensäure-13-Hydroxylase, die die Sequenz gemäß SEQ ID NO: 1 oder SEQ ID NO: 3 umfasst, mindestens eine Substitution einer Aminosäure umfasst, die einer der Aminosäuren an Position 136, 248, 336 oder 403 entspricht, wobei es sich bei der Substitution gegen die Position 136 entsprechende Aminosäure um ein Methionin, Alanin, Phenylalanin, Tryptophan, Glutamin, Histidin oder Threonin handelt, wobei die Positionen unter Bezugnahme auf SEQ ID NO: 1 oder SEQ ID NO: 3 definiert sind und wobei das Polypeptid eine modifizierte KAH-Aktivität im Vergleich mit dem Referenzpolypeptid mit Kaurensäure-13-Hydroxylase-Aktivität gemäß SEQ ID NO: 3 aufweist, wobei bei Überexpression des Polypeptids mit modifizierter Aktivität in einer Wirtszelle die modifizierte Aktivität zu einer Zunahme des Verhältnisses zwischen der Summe von Steviolglycosiden und der Summe von Kaurensäureglycosiden im Vergleich zum Produktionsniveau einer äquivalenten Wirtszelle, die das Referenzpolypeptid überexprimiert, führt, wobei es sich bei der Summe von Steviolglycosiden um die Summe des Produktionsniveaus von Steviol-13-monosid, Steviol-19-monosid, Steviolbiosid, Rubusosid, Rebaudiosid B, Steviosid, Rebaudiosid A, Rebaudiosid E, Rebaudiosid D und Rebaudiosid M handelt, wobei es sich bei der Summe von KA-Glycosiden um die Summe des Produktionsniveaus von KA, KA-19-Monoglucosid, KA-19-Diglucosid und KA-19-Triglucosid handelt, wobei die Positionen im Polypeptid mit Kaurensäure-13-Hydroxylase-Aktivität, die den Aminosäuren an Position 136, 248, 336 oder 403 entsprechen, durch Ausrichten der Aminosäuresequenz des Polypeptids an der Aminosäuresequenz gemäß SEQ ID NO: 1 oder SEQ ID NO: 3 unter Verwendung der EMBOSS-Needle-Ausrichtungsmethode identifiziert werden, bei der EBLOSUM62 als Substitutionsmatrix mit einem *gap-open penalty*-Wert von 10 und einem *gap extension penalty*-Wert von 0,5 verwendet wird, und wobei das Polypeptid eine Aminosäuresequenz mit mindestens 90 %, mindestens 95 %, mindestens 97 %, mindestens 98 % oder mindestens 99 % Sequenzidentität mit SEQ ID NO: 1 oder SEQ ID NO: 3 umfasst.

2. Polypeptid nach Anspruch 1, wobei:
(i) ein Methionin an Position 136 vorhanden ist;
(ii) ein Asparagin an Position 248 vorhanden ist;
(iii) ein Serin an Position 336 vorhanden ist; und/oder
(iv) ein Glycin an Position 403 vorhanden ist,
wobei die Positionen unter Bezugnahme auf SEQ ID NO: 1 oder SEQ ID NO: 3 definiert sind.

3. Polypeptid nach einem der vorhergehenden Ansprüche mit Kaurensäure-13-Hydroxylase-Aktivität, umfassend eine Aminosäuresequenz mit mindestens etwa 95% Sequenzidentität, mindestens 96 %, mindestens 97 %, mindestens 98 % oder mindestens 99 % Sequenzidentität mit einer von SEQ ID NO: 5, 7, 9, 11 oder 13.

4. Nukleinsäure, umfassend eine Sequenz, die ein Polypeptid nach einem der vorhergehenden Ansprüche codiert.

5. Rekombinante Wirtszelle, umfassend eine Nukleinsäure nach Anspruch 4, die gegebenenfalls in der Lage ist, Steviol oder ein Steviolglycosid zu produzieren.

6. Rekombinante Wirtszelle nach Anspruch 5, die eine oder mehrere rekombinante Nukleotidsequenz(en) umfasst, die Folgendes codiert bzw. codieren:
ein Polypeptid mit ent-Copalyldiphosphat-Synthase-Aktivität;
ein Polypeptid mit ent-Kauren-Synthase-Aktivität; und
ein Polypeptid mit ent-Kauren-Oxidase-Aktivität; und gegebenenfalls
ein Polypeptid mit Kaurensäure-13-Hydroxylase-Aktivität,
das sich von einem Polypeptid nach einem der Ansprüche 1 bis 3 unterscheidet.

7. Rekombinante Wirtszelle nach Anspruch 5 oder 6, die eine rekombinante Nukleinsäuresequenz umfasst, die ein Polypeptid mit NADPH-Cytochrom-p450-Reduktase-Aktivität codiert.

8. Rekombinante Wirtszelle nach einem der Ansprüche 5 bis 7, die eine rekombinante Nukleinsäuresequenz umfasst, die ein oder mehrere aus Folgendem codiert:
(i) ein Polypeptid mit UGT74G1-Aktivität;
(ii) ein Polypeptid mit UGT2-Aktivität;
(iii) ein Polypeptid mit UGT85C2-Aktivität; und
(iv) ein Polypeptid mit UGT76G1-Aktivität.

9. Rekombinante Wirtszelle nach einem der Ansprüche 5 bis 8, wobei die Wirtszelle zu einem der Genera *Saccharomyces, Aspergillus, Pichia, Kluyveromyces, Candida, Hansenula, Humicola, Issatchenkia, Trichosporon, Brettanomyces, Pachysolen, Yarrowia, Yamadazyma* oder *Escherichia* gehört, beispielsweise eine *Saccharomyces cerevisiae*-Zelle, eine *Yarrowia lipolytica*-Zelle, eine *Candida krusei*-Zelle, eine *Issatchenkia orientalis*-Zelle oder eine *Escherichia coli*-Zelle.

10. Verfahren zur Herstellung von Steviol oder einem Steviolglycosid, das Fermentieren einer rekombinanten Wirtszelle nach einem der Ansprüche 5 bis 9 in einem geeigneten Fermentationsmedium und gegebenenfalls Gewinnen des Steviols bzw. Steviolglycosids umfasst.

11. Fermentationsbrühe, umfassend ein Steviolglycosid und die rekombinante Wirtszelle nach einem der Ansprüche 5 bis 9.

## Revendications

1. Polypeptide ayant une activité d'acide kaurénoïque 13-hydroxylase (KAH), lequel polypeptide comprend une séquence d'acides aminés qui, lorsqu'elle est alignée avec une acide kaurénoïque 13-hydroxylase comprenant la séquence présentée dans SEQ ID NO: 1 ou SEQ ID NO: 3, comprend au moins une substitution d'un acide aminé correspondant à l'un quelconque des acides aminés aux positions 136, 248, 336, ou 403, où la substitution de l'acide aminé correspondant à la position 136 est une méthionine, alanine, phénylalanine, tryptophane, glutamine, histidine ou thréonine,
lesdites positions étant définies en référence à SEQ ID NO: 1 ou SEQ ID NO: 3 et dans lequel le polypeptide a une activité de KAH modifiée par rapport au polypeptide de référence ayant une activité d'acide kaurénoïque 13-hydroxylase selon SEQ ID NO: 3, dans lequel l'activité modifiée conduit à une augmentation du rapport entre la somme des glycosides de stéviol et la somme des glycosides d'acide kaurénoïque lorsque le polypeptide à activité modifiée est surexprimé dans une cellule hôte par rapport au niveau de production d'une cellule hôte équivalente qui surexprime le polypeptide de référence, où la somme des glycosides de stéviol est la somme du niveau de production de stéviol-13-monoside, stéviol-19-monoside, stéviol bioside, rubusoside, rébaudioside B, stévioside, rébaudioside A, rébaudioside E, rébaudioside D et rébaudioside M, où la somme des KA-glycosides est la somme du niveau de production de KA, KA-19-monoglucoside, KA-19-diglucoside et KA-19-triglucoside, où les positions dans le polypeptide ayant une activité d'acide kaurénoïque 13-hydroxylase correspondant à l'un quelconque des acides aminés aux positions 136, 248, 336 ou 403, sont identifiées par alignement de la séquence d'acides aminés du polypeptide ayant une activité d'acide kaurénoïque 13-hydroxylase avec la séquence d'acides aminés présentée dans SEQ ID NO: 1 ou SEQ ID NO: 3 en utilisant le procédé d'alignement EMBOSS Needle, en utilisant EBLOSUM62 comme une matrice de substitution, avec une pénalité d'ouverture de brèche de 10 et une pénalité d'extension de brèche de 0,5, et dans lequel le polypeptide comprend une séquence d'acides aminés ayant au moins 90 %, au moins 95 %, au moins 97 %, au moins 98 % ou au moins 99 % d'identité de séquence avec SEQ ID NO: 1 ou SEQ ID NO: 3.

2. Polypeptide selon la revendication 1, dans lequel :
(i) une méthionine est présente en position 136 ;
(ii) une asparagine est présente en position 248 ;
(iii) une sérine est présente en position 336 ; et/ou
(iv) une glycine est présente en position 403, lesdites positions étant définies en référence à SEQ ID NO: 1 ou SEQ ID NO: 3.

3. Polypeptide selon l'une quelconque des revendications précédentes ayant une activité d'acide kaurénoïque 13-hydroxylase comprenant une séquence d'acides aminés ayant au moins environ 95 % d'identité de séquence, au moins 96 %, au moins 97 %, au moins 98 % ou au moins 99 % d'identité de séquence avec l'une quelconque parmi les SEQ ID NO: 5, 7, 9, 11 ou 13.

4. Acide nucléique comprenant une séquence codant pour un polypeptide selon l'une quelconque des revendications précédentes.

5. Cellule hôte recombinante comprenant un acide nucléique selon la revendication 4, éventuellement qui est capable de produire du stéviol ou un glycoside de stéviol.

6. Cellule hôte recombinante selon la revendication 5, qui comprend une ou plusieurs séquence(s) nucléotidique(s) recombinante(s) codant pour :
un polypeptide ayant une activité d'ent-copalyl pyrophosphate synthase ;
un polypeptide ayant une activité d'ent-kaurène synthase ; et
un polypeptide ayant une activité d'ent-kaurène oxydase ; et, éventuellement,
un polypeptide ayant une activité d'acide kaurénoïque 13-hydroxylase qui est différent d'un polypeptide selon l'une quelconque des revendications 1 à 3.

7. Cellule hôte recombinante selon la revendication 5 ou 6, qui comprend une séquence d'acide nucléique recombinante codant pour un polypeptide ayant une activité de NADPH-cytochrome p450 réductase.

8. Cellule hôte recombinante selon l'une quelconque des revendications 5 à 7, qui comprend une séquence d'acide nucléique recombinante codant pour un ou plusieurs parmi :
(i) un polypeptide ayant une activité d'UGT74G1 ;
(ii) un polypeptide ayant une activité d'UGT2 ;
(iii) un polypeptide ayant une activité d'UGT85C2 ; et
(iv) un polypeptide ayant une activité d'UGT76G1.

9. Cellule hôte recombinante selon l'une quelconque des revendications 5 à 8, la cellule hôte appartenant à l'un des genres Saccharomyces, Aspergillus, Pichia, Kluyveromyces, Candida, Hansenula, Humicola, Issatchenkia, Trichosporon, Brettanomyces, Pachysolen, Yarrowia, Yamadazyma ou Escherichia, par exemple une cellule de Saccharomyces cerevisiae, une cellule de Yarrowia lipolytica, une cellule de Candida krusei, une cellule d'Issatchenkia orientalis ou une cellule d'Escherichia coli.

10. Procédé pour la préparation de stéviol ou d'un glycoside de stéviol qui comprend la fermentation d'une cellule hôte recombinante selon l'une quelconque des revendications 5 à 9 dans un milieu de fermentation approprié et, éventuellement, la récupération du stéviol ou du glycoside de stéviol.

11. Bouillon de fermentation comprenant un glycoside de stéviol et la cellule hôte recombinante selon l'une quelconque des revendications 5 à 9.
